# EUROPÄISCHE PATENTSCHRIFT

(11) **EP 0 886 678 B1**
(45) Veröffentlichungstag und Bekanntmachung des Hinweises auf die Patenterteilung: **29.06.2005**
(21) Anmeldenummer: 96907487.1
(22) Anmeldetag: 15.03.1996
(51) Int. Cl.: C12N 15/62, C12N 15/65, C12N 15/70, C07K 16/00

(54) **EXPORTSYSTEME FÜR REKOMBINANTE PROTEINE**
EXPORT SYSTEMS FOR RECOMBINANT PROTEINS
SYSTEMES D'EXPORTATION POUR PROTEINES RECOMBINEES

(43) Veröffentlichungstag der Anmeldung: 30.12.1998
(73) Patentinhaber: Max-Planck-Gesellschaft zur Förderung der Wissenschaften e.V. Berlin, 80539 München (DE)
(72) Erfinder: MAURER, Jochen, D-72070 Tübingen (DE); JOSE, Joachim, D-72076 Tübingen (DE); MEYER, Thomas, F., D-72076 Tübingen (DE)
(74) Vertreter: Weiss, Wolfgang, Dipl.-Chem. Dr.
(86) Internationale Anmeldenummer: PCT/EP1996/001130
(87) Internationale Veröffentlichungsnummer: WO 1997/035022

(56) Entgegenhaltungen:
- WO-A-93/10214
- WO-A-95/17509
- VACCINE, Bd. 12, Nr. 6, Mai 1994, Seiten 492-498, XP000651455 RUPPERT, A. ET AL.: "OmpA-FMDV VP1 fusion proteins: production, cell surface exposure and immune responses to the major antigenic domain of foot-and-mouth disease virus"
- EMBO J., Bd. 11, Nr. 6, 1992, Seiten 2327-2335, XP002028468 KLAUSER, T. ET AL.: "Selective extracellular release of cholera toxin B subunit by Escherichia coli: dissection of Neisseria Iga -mediated outer membrane transport"
- MOLECULAR MICROBIOLOGY, Bd. 18, Nr. 2, 1995, Seiten 377-382, XP000651438 JOSE, J. ET AL.: "MicroCorrespondence: Common structural featues of IgA1 protease-like outer membrane protein autotransporters"
- BIO-TECHNOLOGY, Bd. 14, Nr. 2, Februar 1996, Seiten 203-208, XP002028469 CORNELIS, P. ET AL.: "Development of new cloning vectors for the production of immunogenic outer membrane fusion proteins in Escherichia coli"
- MOLECULAR MICROBIOLOGY, Bd. 17, Nr. 1, 1995, Seiten 123-135, XP000651454 BENJELLOUN-TOUIMI, Z. ET AL.: "SepA, the major extracellular protein of Shigella flexneri: autonomous secretion and involvement in tissue invasion"
- J.BACTERIOLOGY, Bd. 179, Nr. 3, Februar 1997, Seiten 794-804, XP000651378 MAURER, J. ET AL.: "Autodisplay: One-Component System for efficient surface display and release of soluble recombinant proteins from Escherichia coli"

## Beschreibung

Die vorliegende Erfindung betrifft Vektoren, Wirt-Vektorkombinationen und Verfahren zur Herstellung von stabilen Fusionsproteinen bestehend aus einem Trägerprotein und einem Passagierprotein, wobei die Expression der Fusionsproteine zur Exposition der Passagierdomänen auf der Oberfläche von Bakterienzellen, insbesondere Escherichia coli Zellen führt. Bei Bedarf können die Passagierdomänen durch Proteasen, z.B. durch ausgewählte Wirtsfaktoren, wie etwa OmpT in das Milieu freigesetzt werden.

Die vorliegende Erfindung betrifft darüberhinaus die Verwendung von Trägerproteinen oder Trägerproteinanteilen aus natürlichen Proteinen, die als Aminosäuresequenzen in Datenbanken oder Dateien vorliegen und entsprechend ihrer Eigenschaften Autotransporter genannt werden.

Die vorliegende Erfindung ermöglicht Verfahren zur Identifizierung und Selektion von Bakterien, die mindestens ein Passagierprotein auf ihrer Oberfläche mit definierter Affinität zu einem Bindungspartner exprimieren, sowie deren Verwendung für diagnostische Zwecke. Insbesondere erlaubt das erfindungsgemäße Verfahren die Expression von Peptid-Bibliotheken auf der Oberfläche von Bakterienzellen, mit deren Hilfe beispielsweise die Bestimmung des Liganden mit der höchsten Affinität bei Antikörpern, MHC-Molekülen oder anderen Komponenten des Immunsystems möglich ist.

Darüberhinaus ermöglicht das erfindungsgemäße Verfahren die Produktion von Fusionsproteinen, die sich aus Anteilen schwerer und leichter Antikörperdomänen und einem Autotransporter zusammensetzen, und deren Transport durch die bakterielle Zellhülle. In einer spezifischen Ausführungsform wird letztlich die zielgerichtete Varianz von bindungsaktiven rekombinanten Antikörpern und deren funktionelle Präsentation auf der Zelloberfläche von Escherichia coli möglich.

Generell erlaubt das erfindungsgemäße Verfahren die Expression von rekombinanten Proteinen, die Rezeptoren oder Liganden sein können, auf der Bakterienoberfläche, sowie die Selektion aufgrund der Bindungsaffinität zu einem Bindungspartner, womit eine damit einhergehende Selektion eines klonalen Produzenten möglich wird.

Erfindungsgemäß ist auch eine Verwendung von Bakterien, die Proteinfusionen auf der Zelloberfläche exprimieren und die gebunden an ein Trägermaterial oder in Lösung vorliegen, zur gezielten Anreicherung oder Reinigung eines zu den Oberflächen-exponierten Proteindomänen Affinität zeigenden Bindungspartners. Darüberhinaus betrifft die vorliegende Erfindung auch die Oberflächenexpression von Enzymen oder anderen Proteinen mit biologisch, chemisch oder technisch relevanten Eigenschaften, sowie, im Bedarfsfalle, deren gezielte Freisetzung in das umgebende Milieu.

Die Oberflächenexposition von rekombinanten Proteinen auf Bakterienzellen ist ein Verfahren mit einer Vielzahl von möglichen mikrobiologischen, molekularbiologischen, immunologischen oder technischen Anwendungen. Durch Produktion von rekombinanten Proteinen auf diese Art und Weise werden ihre Eigenschaften, z.B. Bindungsaffinitäten oder enzymatische Aktivitaten (Francisco et al., Bio.Technology 11 (1993) 491 - 495) zugänglich, ohne daß ein weiterer Schritt, wie beispielsweise der Aufschluß der Produzentenzelle notwendig ist. Da nur eine limitierte Anzahl von Faktoren natürlicherweise auf der Baktereinoberfläche exprimiert werden, ist zusätzlich eine spezifische Anreicherung des rekombinanten Proteins im Vergleich zu einer cytosolischen Produktion gegeben. Ein weiterer wesentlicher Vorteil ist, daß man mit den gleichen Methoden, mit denen man das gesuchte rekombinante Protein selektioniert, auch den Produzenten dieses Proteins, eine Bakterienzelle isolieren kann, und somit einen klonalen Produzenten erhält, der dauerhaft gelagert, stabil reproduziert und in großem Maßstab vermehrt werden kann.

Für die Präsentation rekombinanter Proteine auf der Zelloberfläche wurden bisher verschiedene Systeme verwendet, die jedoch ausnahmslos auch natürlicherweise für den Transport oder die Sekretion bakterieller Oberflächenproteine dienen (Little et al., TIBTECH 11 (1993), 3 - 5). Sinnvollerweise wurde dabei die DNA-Region, die natürlicherweise für das zu transportierende Protein, den Passagier, kodiert, ersetzt oder ergänzt durch die kodierende DNA-Region des gewünschten rekombinanten Proteines, wobei jedoch die kodierenden Bereiche der für den Transport verantwortlichen Proteindomänen, die Trägerprcteine, in der Regel unverändert blieben. Daraus wird deutlich, daß Systeme, in denen Passagier- und Trägerkomponente unmittelbar benachbart oder in einem Gen kodiert vorliegen, sogenannte Ein-Komponenten-Systeme, einen erheblichen Vorteil gegenüber Systemen mit mehreren, unabhängigen Komponenten (Gentschev et al., Behring Inst. Mitt. 95 (1994) 57 - 66) haben, insbesondere bei der Herstellung von universell verwendbaren Vektoren, die neben der Eigenschaft zur stabilen Replikation, einem oder mehreren Selektionsmarkern, den für den Transport notwendigen Proteindomänen, auch eine Insertionsstelle für das den Passagier kodierende DNA-Fragment enthalten müssen. Als Trägerproteine in vielen bisher verwendeten Ein-Komponenten-Systemen wurden Proteine der äußeren Membran von E. coli verwendet. Dazu gehören unter anderem LamB (Charbit et al., Gene 70 (1988), 181 - 189), PhoE (Agterberg et al., Gene 59 (1987) 145 - 150) oder OmpA (Francisco et al., Proc. Natl. Acad. Sci (1992), 2713 - 2717), deren Verwendung jedoch Nachteile bergen. So können zusätzliche Proteinsequenzen nur in oberflächenexponierten Schleifen integriert werden, was einmal zu fixierten amino- und carboxyterminalen Enden an den einrahmenden Trägerproteinsequenzen führt und zum anderen sich limitierend auf die Länge der einzubringenden Sequenzen auswirkt. Die Verwendung des Peptidoglykan assoziierten Lipoproteins (PAL) als Trägerprotein führt zwar zum Transport zur äußeren Membran, eine Präsentation nativer Proteinsequenzen auf der Oberfläche von E.coli ist damit jedoch nicht möglich (Fuchs et al., Bio.Technology 9 (1991), 1369 - 1372). Eine Oberflächenexpression größerer Proteine ist möglich unter Verwendung einer Fusion aus OmpA und Lpp als Trägerproteinanteil, an deren Carboxylende die Passagierproteinsequenzen angehängt werden (Francisco et al., Proc. Natl. Acad. Sci (1992), 2713 - 2717). Dabei ist als Nachteil in Kauf zu nehmen, daß die Fixierung des N-Terminus des Passagiers eine korrekte Faltung oder Funktion verhindern kann.

Weiterhin sind sogenannte Autotransporter enthaltende Proteine bekannt, eine Familie von sekretierten Proteinen in Gram-negativen Bakterien. In der Publikation Jose et al. (Mol.Microbiol. 18 (1995), 377-382) werden einige Beispiele für solche Autotransporterproteine genannt. Diese Proteine enthalten eine Proteindomäne, die den Transport einer N-terminalen angehängten Proteindomäne durch eine aus β-Faltblattstrukturen gebildete Porenstruktur in der Außenmembran Gram-negativer Bakterien ermöglicht. Die Autotransporter enthaltenden Proteine werden als sogenanntes Poly-Protein-Vorläufermolekül synthetisiert. Der typische Aufbau eines solchen Vorläuferproteins ist dreigeteilt. Am N-Terminus befindet sich eine Signalsequenz, die verantwortlich ist für den Transport durch die innere Membran, unter Inanspruchnahme des im Wirt vorhandenen Sec-Transportapparates und die dabei abgetrennt wird. Daran schließt sich die zu sekretierende Proteindomäne an, gefolgt von einer C-terminalen Helferdomäne, die eine Pore in der Außenmembran bildet, wodurch die N-terminal angehängte zu sekretierende Proteindomäne an die Oberfläche transloziert wird. Dort bleibt diese in Abhängigkeit ihrer auszuübenden Funktion mit dem nun als Membrananker dienenden Helfer an der Bakterienoberfläche verbunden oder wird durch proteolytische Aktivität abgetrennt, wobei diese proteolytische Aktivität der zu sekretierenden Proteindomäne innewohnen kann oder eine vom Wirt ausgehende Eigenschaft sein kann oder eine externe/gezielt zugesetzte Aktivität (z.B. Thrombin, IgA-Protease) sein kann. Die Sektretion heterologer Polypeptide bzw. Proteine unter Verwendung eines auf einem Autotransporter basierenden Expressionssystems ist bekannt. So ist beispielsweise aus EP-A-0 254 090 oder der Publikation Klauser et al. (EMBO J. 11 (1992), 2327-2335) bekannt, daß die Helferdomäne der IgA-Protease aus N. gonorrhoeae heterologe Polypeptide als Passagierdomänen in den heterologen Bakterienstämmen E.coli und Salmonella typhimurium exprimieren kann.

Weiterhin ist der extrazelluläre Transport des Proteins VirG von Shigella bei Suzuki et al. (J.Biol.Chem. 270 (1995), 30874-30880) beschrieben. Bei diesem Protein handelt es sich ebenfalls um einen IgA-Protease-ähnlichen Autotransporter, der zur Expression fremder Polypeptide wie etwa MalE und PhoA in der Lage ist, die kovalent an den N-Terminus der Autotransporterdomäne von VirG verknüpft wurden. Weiterhin wird in der Arbeit Shimada et al. (J.Biochem. 116 (1994), 327-334) der extrazelluläre Transport eines heterologen Polypeptids, nämlich Pseudoazurin aus A. faecales in E.coli unter Verwendung der Autotransporterdomäne der Serinprotease von S. marcescens beschrieben.

Bei den im Stand der Technik beschriebenen Verfahren zur Herstellung zur Expression heterologer Passagierproteine mit Hilfe von Autotransportersystemen wurden jedoch erhebliche Nachteile festgestellt. So treten bei Verwendung der Transporter- bzw. Helferdomäne der IgA-Protease aus N. gonorrhoeae in E.coli als Wirtstamm häufig erhebliche Kompatibilitätsprobleme auf. Zu starke Expression führt zur Zell-Lyse oder die Bakterien zeigen ein vermindertes Wachstum auch bei mittlerer Expression, was in beiden Fällen zu einer erheblich verminderten Fusionsproteinausbeute führt und auf Schwächen in der Stabilität des Systems hinweist. Der vorliegenden Erfindung lag also das technische Problem zugrunde, Trägerproteine bereitzustellen, die insbesondere bei Verwendung von E.coli als Wirtsstamm nicht zu diesen Nachteilen führen, da aus vielerlei Gründen E.coli als Wirtsstamm gegenüber z.B. Neisseria gonorrhoeae vorzuziehen ist. Zum einen lassen sich E.coli Stämme mit rekombinanter DNA schon in einfachen Laboratorien der Sicherheitsstufe 1 anziehen. Darüberhinaus wird bereits mit E.coli Stämmen in der kommerziellen Produktion rekombinanter Proteine gearbeitet. Dies bedeutet einen erheblichen Vorteil im Umgang und in der Handhabung von rekombinanten E.coli Stämmen im Vergleich zu anderen Wirtsstämmen. Darüberhinaus gibt es von E.coli bereits eine große Anzahl genau charakterisierter Mutantenstämme, die in Abhängigkeit der gewünschten Anwendung eine Auswahl des Wirtsstammes zulassen.

Diese Aufgabe wird gelöst durch ein Verfahren zur Präsentation von Peptiden oder/und Polypeptiden auf der Oberfläche von Gram-negativen Wirtsbakterien, wobei man,
(a) ein Wirtsbakterium bereitstellt, das transformiert mit einem Vektor, auf dem in operativer Verknüpfung mit einem Promotor eine fusionierte Nukleinsäuresequenz lokalisiert ist, umfassend:
   (i) einen Signalpeptid-kodierenden Nukleinsäureabschnitt,
   (ii) einen für das zu präsentierende Passagierpeptid oder/und Passagierpolypeptid kodierenden Nukleinsäureabschnitt,
   (iii) gegebenenfalls einen für eine Proteaseerkennungsstelle kodierenden Nukleinsäureabschnitt,
   (iv) einen für einen Transmembranlinker kodierenden Nukleinsäureabschnitt und
   (v) einen für eine Transporterdomäne eines Autotransporters kodierenden Nukleinsäureabschnitt; und
(b) das Wirtsbakterium unter Bedingungen kultiviert, bei denen eine Expression der fusionierten Nukleinsäuresequenz und eine Präsentation des von dem Nukleinsäureabschnitt (ii) kodierten Peptids oder Polypeptids an der Oberfläche des Wirtsbakteriums erfolgt,
dadurch gekennzeichnet, daß der Nukleinsäureabschnitt (ii) gegenüber dem für die Transporterdomäne (v) kodierenden Nukleinsäureabschnitt heterolog und das Wirtsbakterium gegenüber dem für die Transporterdomäne (v) kodierenden Nukleinsäureabschnitt homolog ist.

Durch die Verwendung eines Wirtsbakteriums, welches gegenüber dem für die Transporterdomäne kodierenden Nukleinsäureabschnitt homolog ist, kann überraschenderweise eine gegenüber dem Stand der Technik deutlich verbesserte Oberflächenpräsentation von Peptiden oder/und Polypeptiden, insbesondere auch von kurzen synthetischen Peptiden mit einer Länge von vorzugsweise 4 - 50 Aminosäuren bzw. von eukaryontischen Polypeptiden erreicht werden.

Beim erfindungsgemäßen Verfahren wird ein Wirtsbakterium bereitgestellt, das mit einem bzw. mit mehreren kompatiblen rekombinanten Vektoren transformiert ist. Ein solcher Vektor enthält in operativer Verknüpfung mit einem Promotor und gegebenenfalls weiteren für die Expression erforderlichen Sequenzen eine fusionierte Nukleinsäuresequenz. Diese fusionierte Nukleinsäuresequenz umfaßt (i) einen Signalpeptid-kodierenden Abschnitt, vorzugsweise einen Abschnitt, der für ein Gram-negatives Signalpeptid kodiert, welches den Durchtritt durch die innere Membran in das Periplasma ermöglicht. Weiterhin umfaßt die fusionierte Nukleinsäuresequenz (ii) einen für das zu präsentierende Passagierpeptid bzw. Polypeptid kodierenden Abschnitt. Gegebenenfalls befindet sich 3'seitig von diesem Abschnitt (iii) ein für eine Proteaseerkennungsstelle kodierender Nukleinsäureabschnitt. Beispiele für geeignete Proteaseerkennungsstellen sind Erkennungsstellen für intrinsische, d.h. natürlicherweise in der Wirtszelle vorhandene oder extern zugeführte Proteasen. Beispiele für extern zugeführte Proteasen sind die IgA-Protease (vgl. z.B.

EP-A-0 254 090), Thrombin oder Faktor X. Beispiele für intrinsische Proteasen sind OmpT, OmpK oder Protease X. 3'-seitig von diesem Abschnitt befindet sich (iv) ein für einen Transmembranlinker kodierender Nukleinsäureabschnitt, der eine Präsentation des von Abschnitt (iii) kodierten Peptids oder Polypeptids auf der Außenseite der äußeren Membran des Wirtsbakteriums ermöglicht. 3'-seitig dieses Abschnitts ist ein für eine Transporterdomäne eines Autotransporters kodierender Nukleinsäureabschnitt.

Besonders bevorzugt werden Transmembranlinkerdomänen verwendet, die homolog bezüglich des Autotransporters sind, d.h. die Transmembranlinkerdomänen werden von Nukleinsäureabschnitten direkt 5'-seitig der Autotransporterdomänen kodiert. Die Länge der Transmembranlinker ist vorzugsweise 30 - 160 Aminosäuren.

Die Transporterdomäne ist in der Lage, in der Außenmembran des Wirtsbakteriums ein sogenanntes β-Faß auszubilden. Das β-Faß besteht aus einer geraden Anzahl antiparalleler, amphipathischer, β-Faltblätter. Diese Struktur besitzt wie andere Proteine der Außenmembran Gram-negativer Bakterien am C-Terminus eine aromatische Aminosäure wie Phenylalanin oder Tryptophan. Darauf folgen abwechselnd geladene (polare) und ungeladene (hydrophobe) Aminosäuren, eine Struktur, die eine Rolle bei der Faltung mit der Membran zu spielen scheint. Die Anzahl und Lage der amphipathischen, β-Faltblätter lassen sich mit Hilfe eines geeigneten Computerprogrammes identifizieren und mit Hilfe von Analogien zu den Außenmembranporinen, von denen die Kristallstruktur bekannt ist (Cowan et al., Nature 358 (1992) 727 - 733), zur Konstruktion eines Modells der Faßstruktur verwenden. Vorzugsweise ist die Faßstruktur aufgebaut wie folgt: 9-14, insbesondere ca. 12 Aminosäuren (AS) für einen Membrandurchgang; keine oder eine minimale Anzahl geladener AS zeigen in einem β-Faltblatt nach außen; kleine oder gar keine Schleifen zeigen nach innen, gegebenenfalls zeigen große oder sehr große Schleifen nach außen; das β-Faß setzt sich aus 12, 14, 16 oder 18, insbesondere aus 14 antiparallelen β-Faltblättern zusammen.

Ausgehend von dem Modell des Fasses kann nun der für den Selbsttransport durch die Außenmembran notwendige Bereich festgelegt werden und mit einem Signalpeptid und einer Passagierdomäne auf genetischer Ebene verknüpft werden. Die Expression dieses Konstruktes ermöglicht dann den Transport des Passagierproteins zur Bakterienoberfläche, wobei das Signalpeptid ursprünglich von dem Passagier oder einem anderen Protein stammen kann. Dabei muß berücksichtigt werden, daß zugehörig zu dem β-Faß eine in der Länge und Sequenz geeignete Linkerregion mit verknüpft wird, die durch die gebildete Pore durchreicht und dafür sorgt, daß die Passagierdomänen vollständig an der Oberfläche exponiert sind.

Ein wesentliches Merkmal des erfindungsgemäßen Verfahrens ist, daß das Wirtsbakterium gegenüber dem für die Transporterdomäne kodierenden Nukleinsäureabschnitt homolog ist, d.h. die Wirtszelle und die Transporterdomäne werden aus homologen Familien, z.B. Enterobakterien, vorzugsweise aus homologen Gattungen, z.B. Escherichia, Salmonella oder Helicobacter, besonders bevorzugt aus homologen Spezies, z.B. Escherichia coli, Salmonella typhimurium ausgewählt. Besonders bevorzugt werden Salmonella oder E.coli als Wirtsbakterium und eine ebenfalls aus Salmonella oder E.coli stammende Transporterdomäne oder eine Variante davon verwendet.

Als besonders geeigneter E.coli Wirtsstamm sei hier der Stamm JK321 (DSM 8860) genannt, der ompT⁻, dsbA⁻ ist und den genetischen Marker fpt trägt, was zu einer stabilen Oberflächenexpression auch großer Proteine, wie z.B. der Vₕ-Kette eines Antikörpers mit Hilfe des igaß-Helferproteins führt.

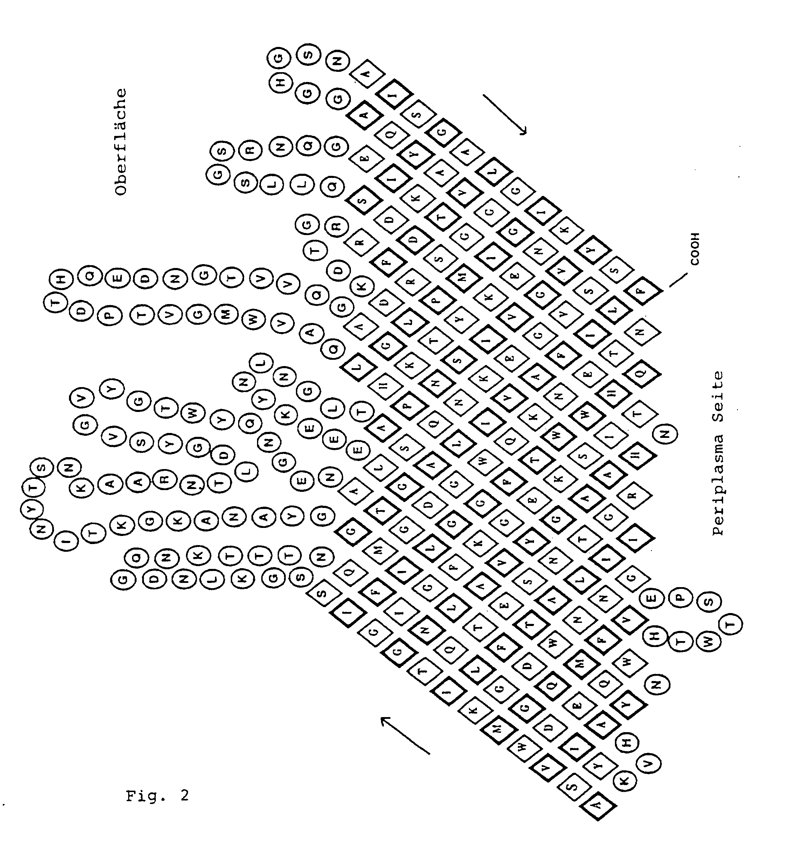

In einer bevorzugten Ausführungsform betrifft die vorliegende Erfindung daher ein Trägerprotein, das eine Autotransporterfunktion ausübt, und eine Oberflächenexposition von rekombinanten Proteine in Escherichia coli mit hoher Ausbeute ermöglicht. In einem typischen Beispiel handelt es sich dabei um den Autotransporter des "adhesin-involved-in-diffuse-adherence" (AIDA-I) aus E.coli (Benz und Schmidt, Infect. Immun. 57 (1989), 1506 - 1511). Die Transporterdomäne des AIDA-I-Proteins ist in Fig. 2 dargestellt. Neben dieser spezifischen Sequenz können auch Varianten davon verwendet werden, die beispielsweise durch Veränderung der Aminosäuresequenz in den nicht am Membrandurchgang beteiligten Schleifenstrukturen erzeugt werden können. Gegebenenfalls können die für die Oberflächenschleifen kodierenden Nukleinsäureabschnitte auch vollständig deletiert werden.

Auch innerhalb der amphipatischen β-Faltblattstrukturen können konservative Aminosäureaustausche, d.h. der Austausch einer hydrophilen durch eine andere hydrophile Aminosäure oder/und der Austausch einer hydrophoben durch eine andere hydrophobe Aminosäure vorgenommen werden.
Eine Variante hat eine Homologie von mindestens 80 % und insbesondere mindestens 90 % zu der in Fig. 2 angegebenen Sequenz der AIDA-I Autotransporterdomäne zumindest im Bereich der β-Faltblattstrukturen.

In einem weiteren typischen Beispiel handelt es sich bei dem verwendeten Autotransporter um den des SepA-Proteins aus Shigella flexneri (Benjellou-Touimi et al., Mol. Micobiol 17 (1995) 123 - 135) oder eine Variante davon. In einem weiteren typischen Beispiel handelt es sich um den Autotransporter des IcsA-Proteins aus Shigella flexneri (Goldberg et al., J.Bacteriol 175 (1993), 2189-2196) oder des Tsh-Proteins aus E.coli (Provence et al., Infect.Immun 62 (1994), 1369-1380). In einem weiteren typischen Beispiel handelt es sich um den Autotransporter des Hsr-Proteins aus Helicobacter mustelae (O'Toole et al., Mol.Microbiol. 11 (1994), 349-361), des Prn-Proteins aus Bordetella ssp. (Charles et al., Proc.Natl. Acad.Sci USA 86 (1989), 3554-3558; Li et al., J.Gen.Microbiol. 138 (1992), 1697-1705), des Ssp-Proteins aus Serratia marcescens (z.B. Yanagida et al., J.Bacteriol. 166 (1986), 937-944 oder Genbank-Accessionnr. X59719, D78380), des Hap-Proteins aus Haemophilus influenzae (StGeme et al., Mol. Microbiol. 14 (1994), 217-233), des BrkA-Proteins aus Bordetella pertussis (Fernandez und Weiss, Infect.Immunol. 62 (1994), 4727-4738), des VacA-Proteins aus Helicobacter pylori (Schmitt und Haas, Mol.Microbiol. 12 (1994), 307-319) oder verschiedener Proteine aus Rickettsien (z.B. 190kDa Zelloberflächenantigene, Genbank-Accessionnr. M31227; SpaP, Carl et al., Proc.Natl.Acad.Sci. USA 87 (1990), 8237-8241; rOmpB, Gilmore et al., Mol.Microbiol. 5 (1991), 2361-2370 und Slp T, Hahn et al., Gene 133 (1993), 129-133) bzw. eine - wie vorstehend definierte - Variante davon.

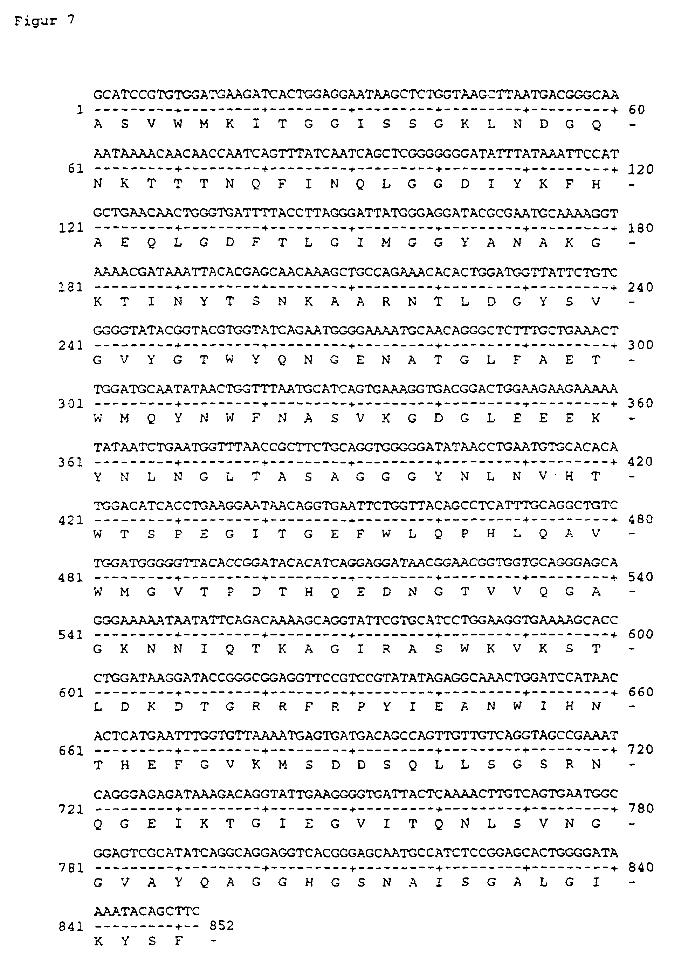

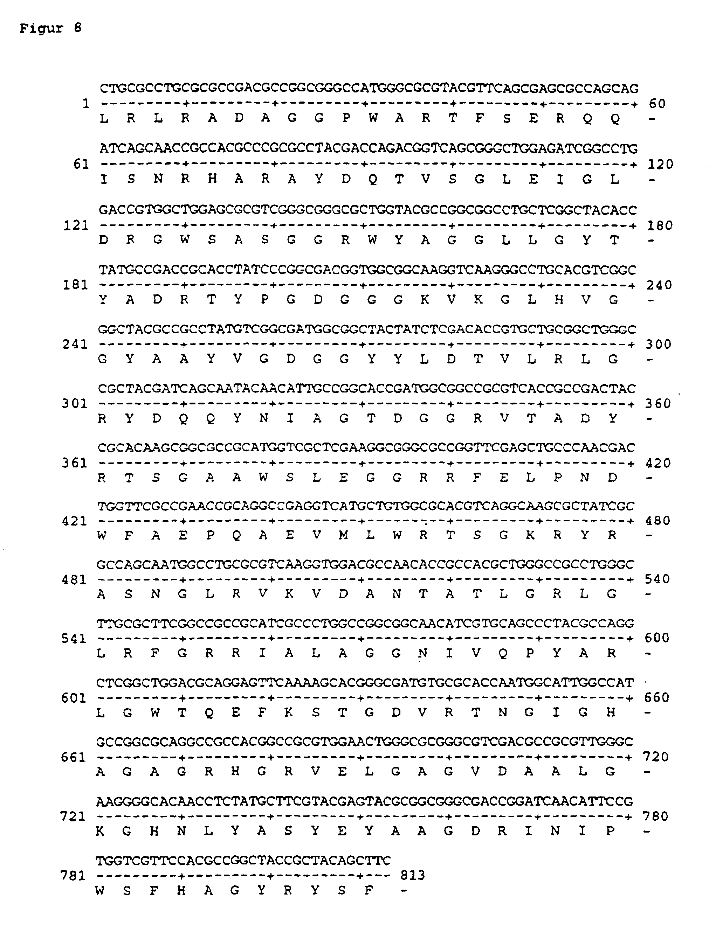

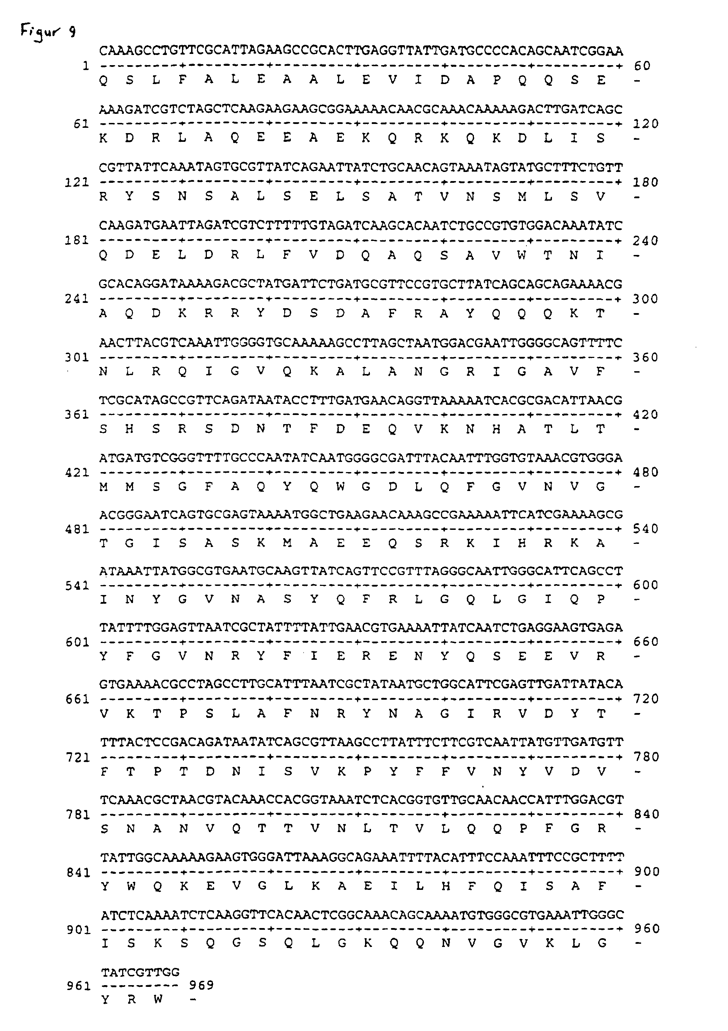

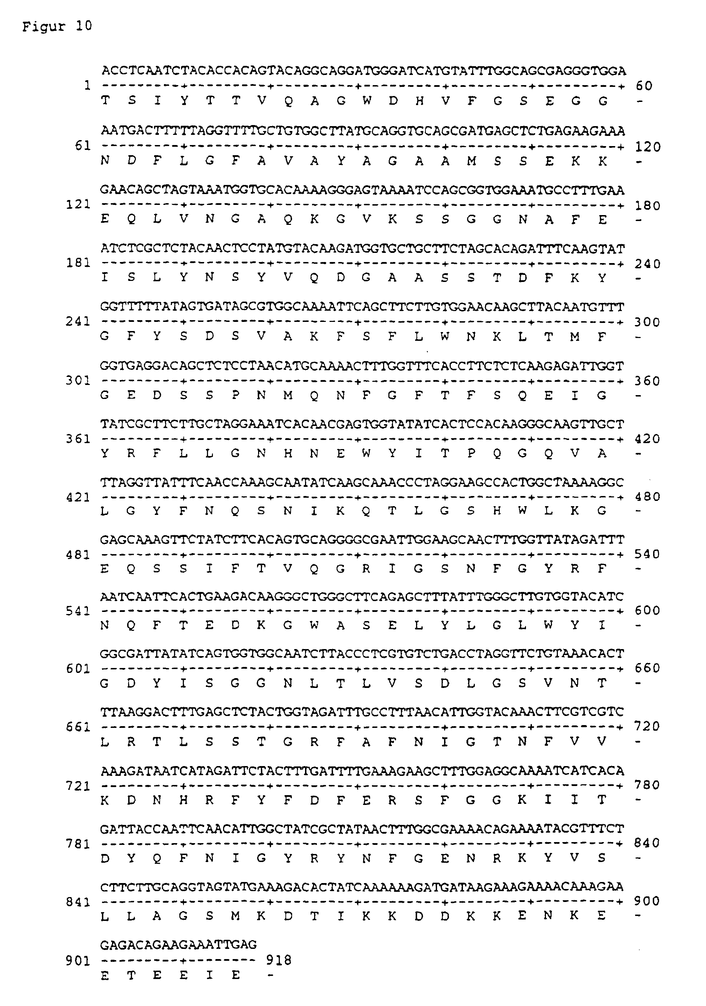

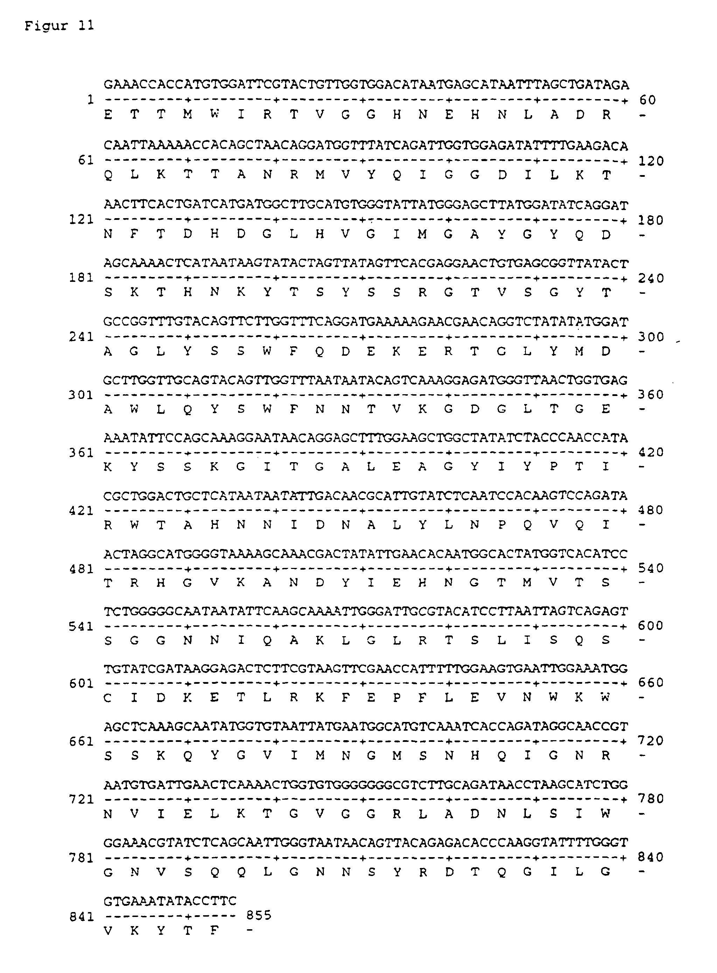

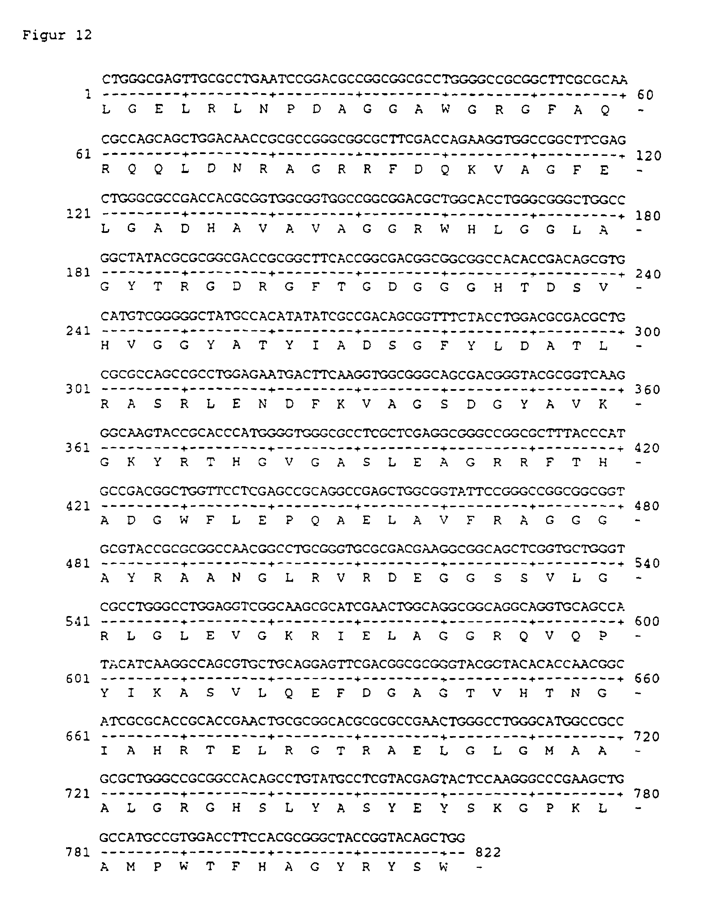

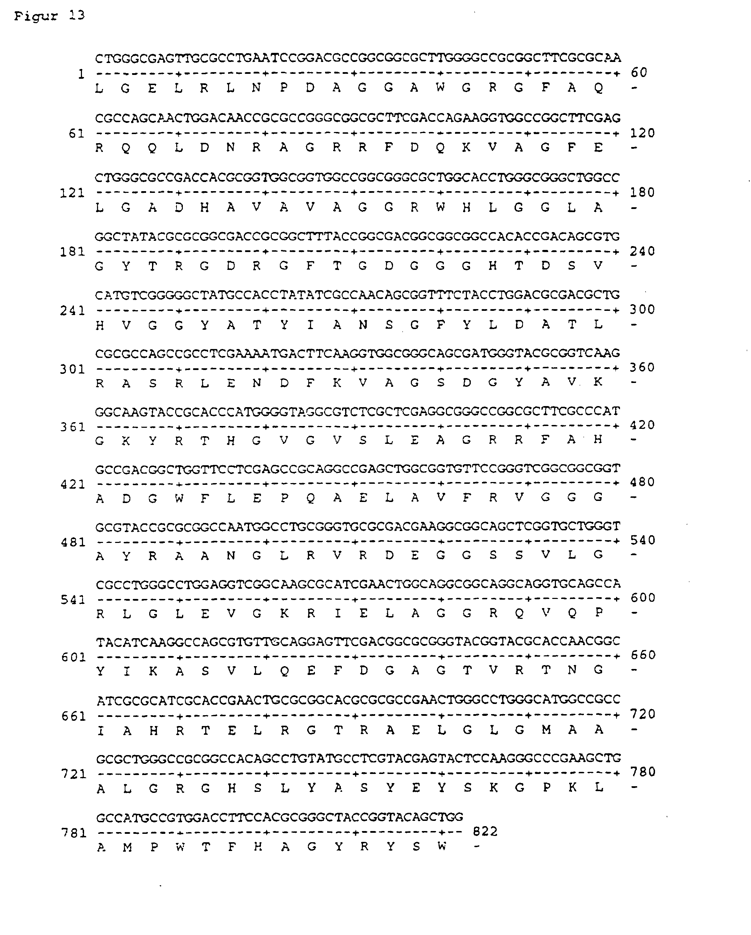

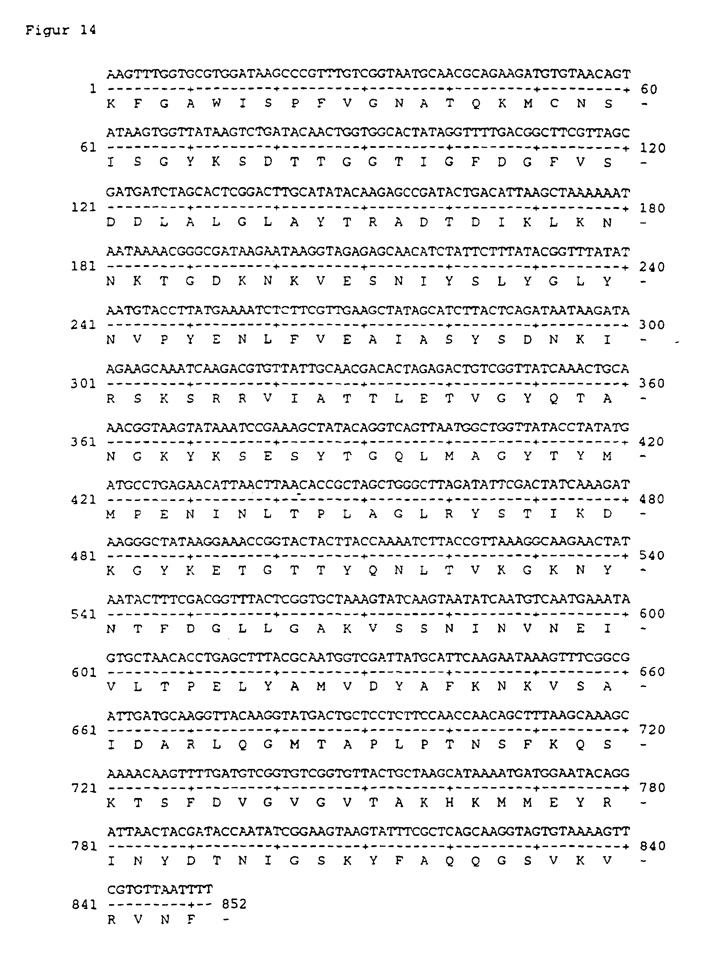

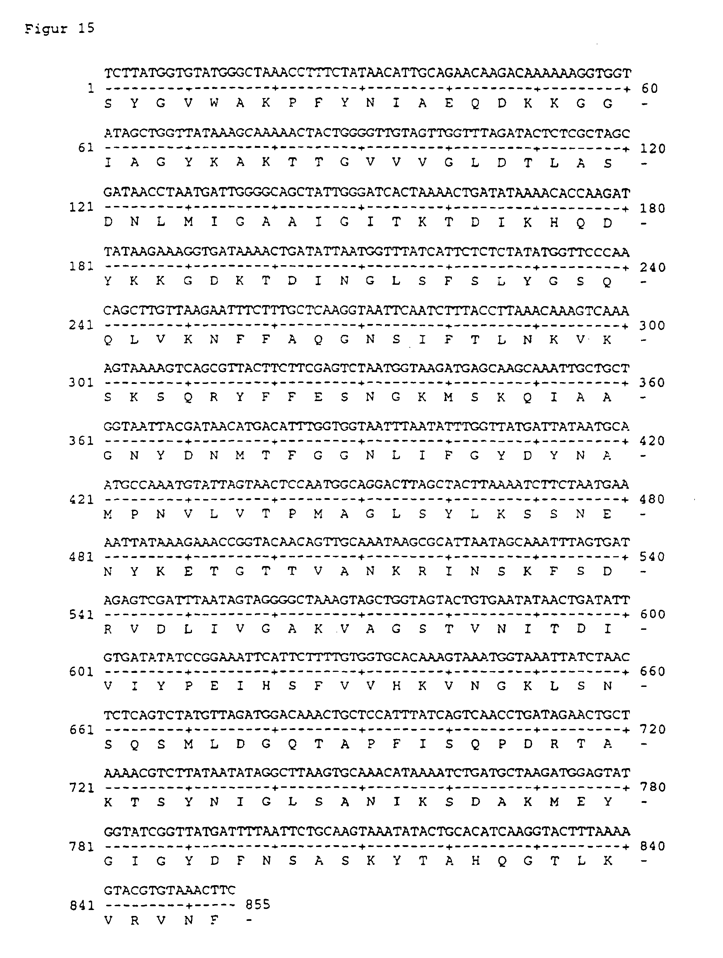

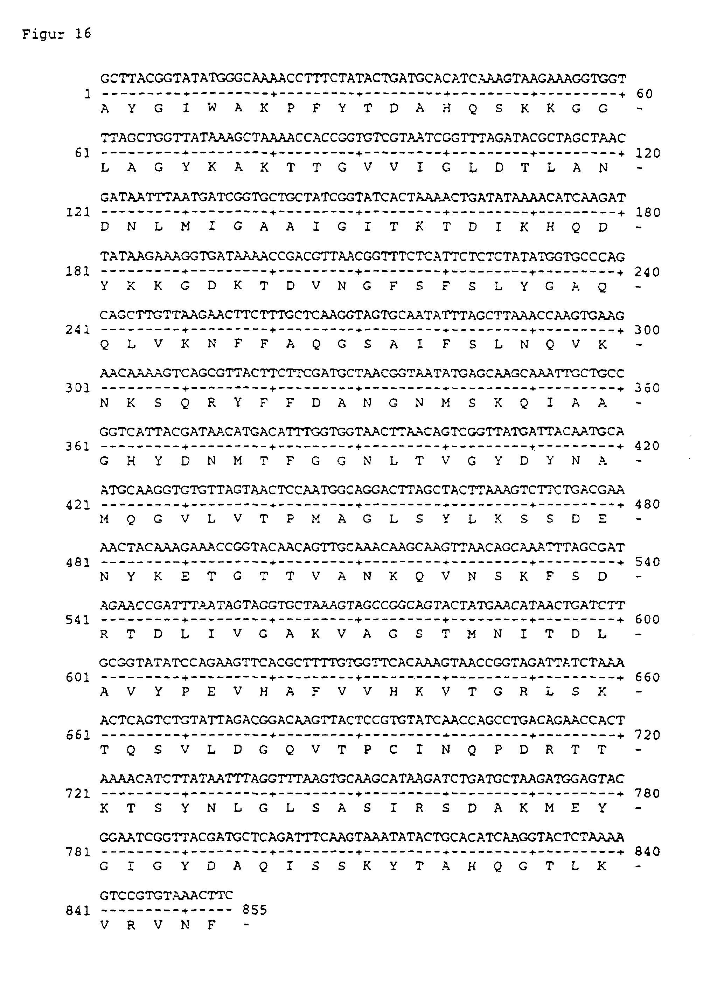

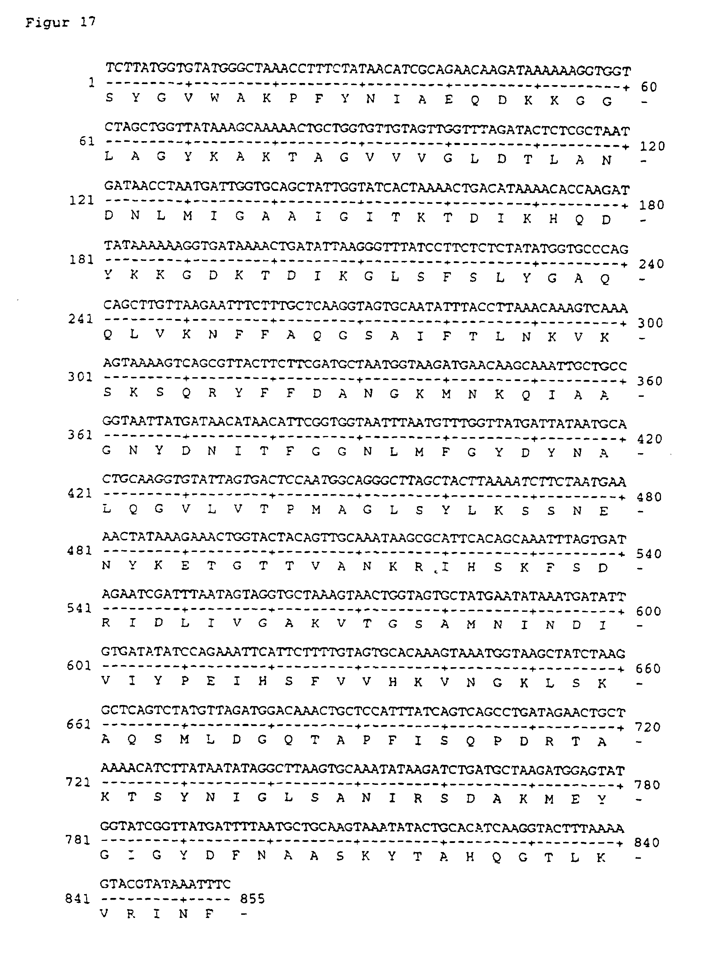

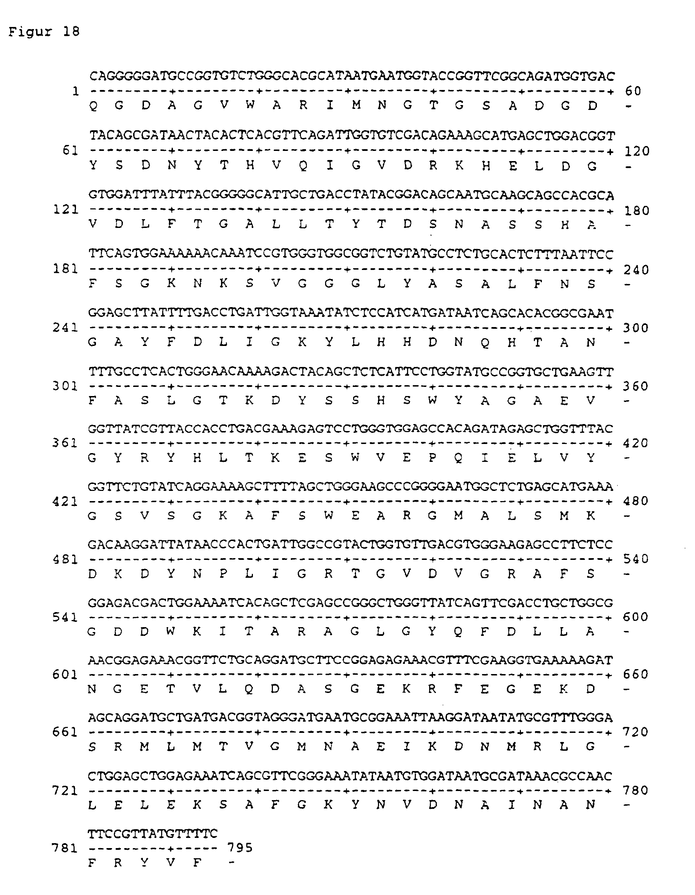

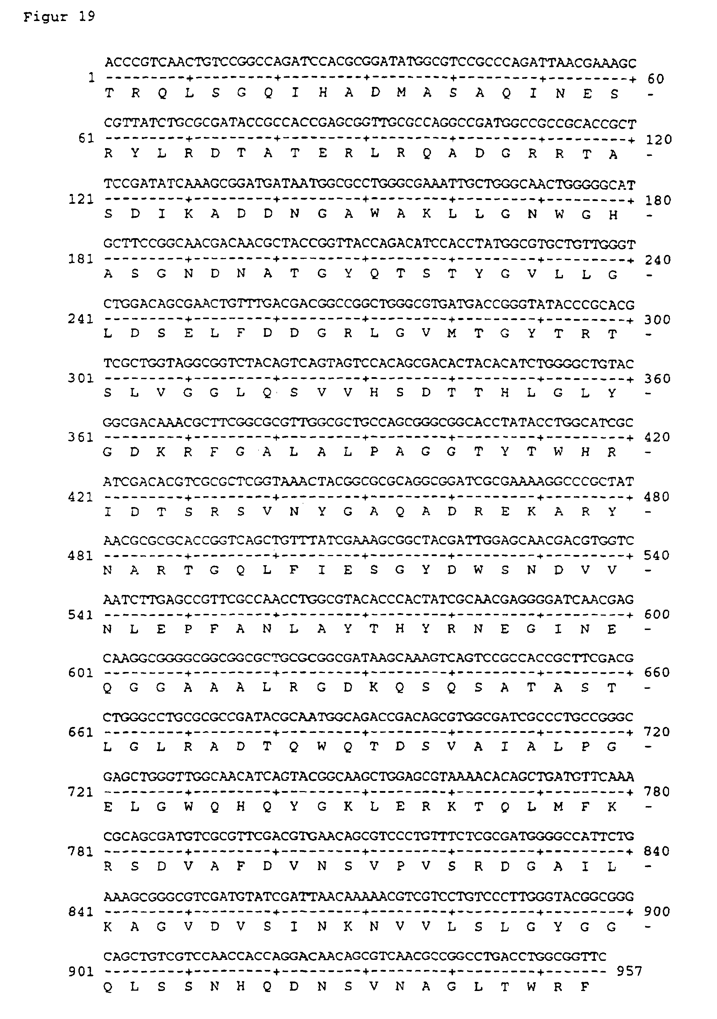

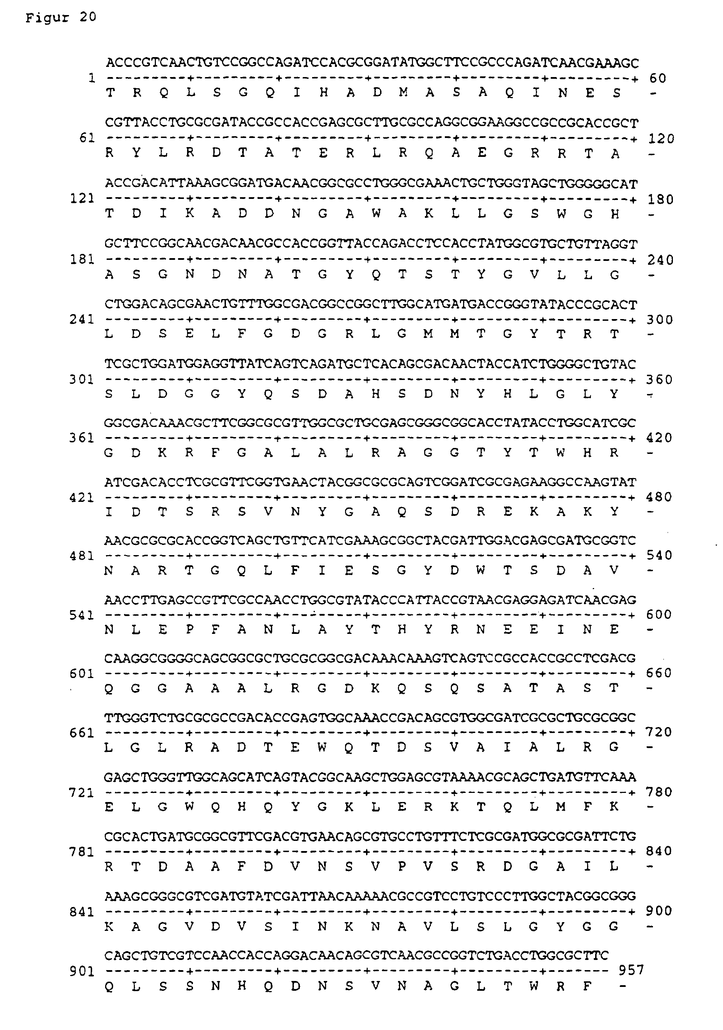

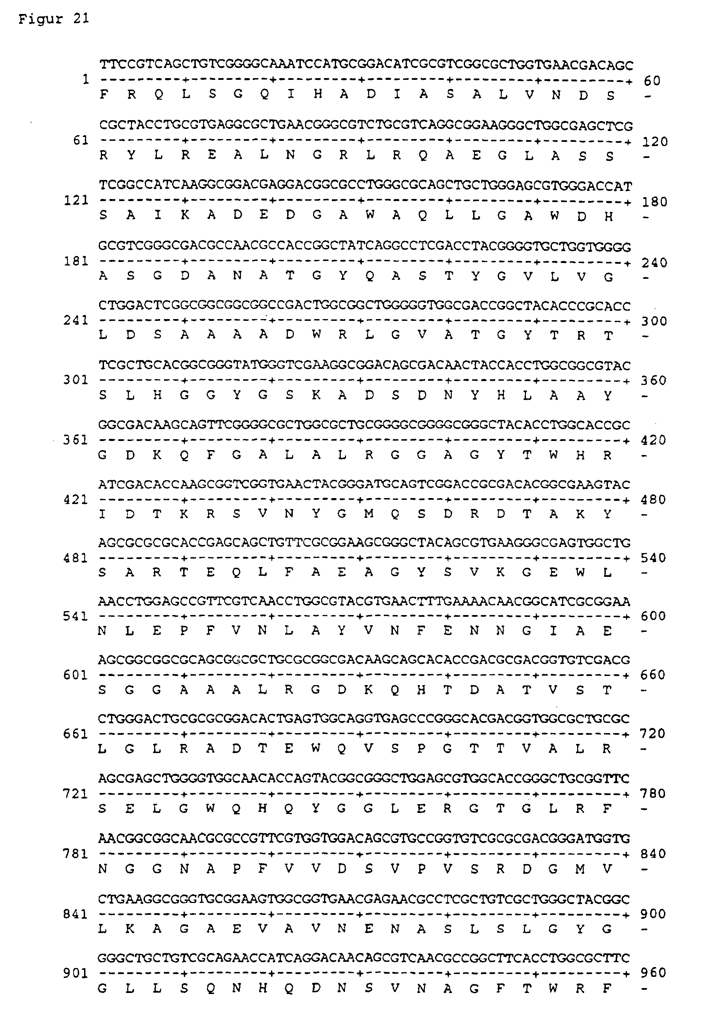

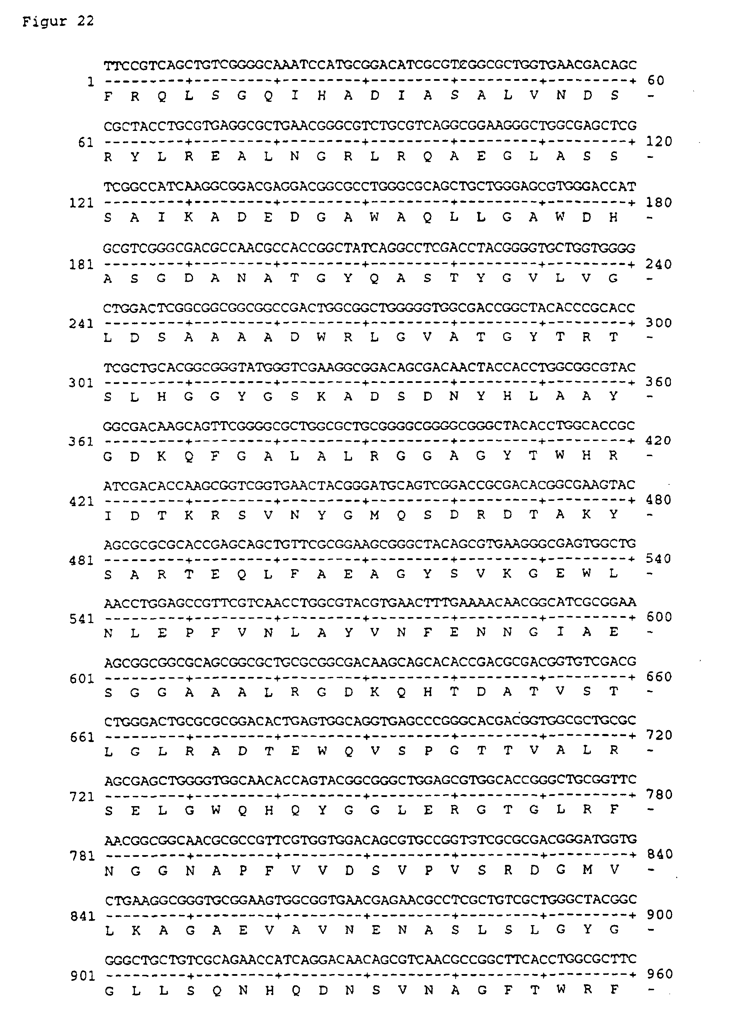

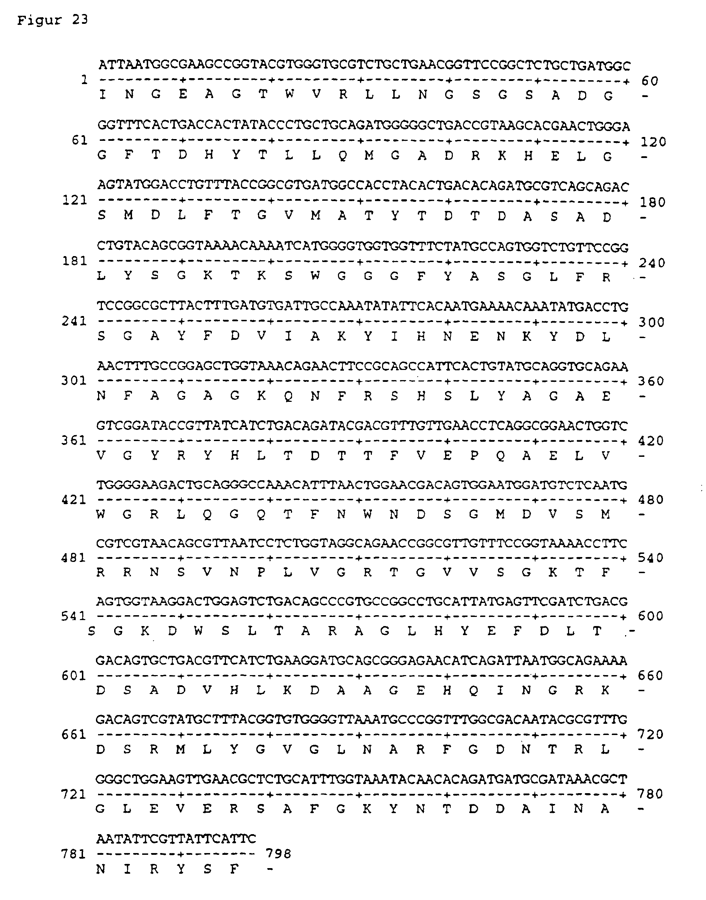

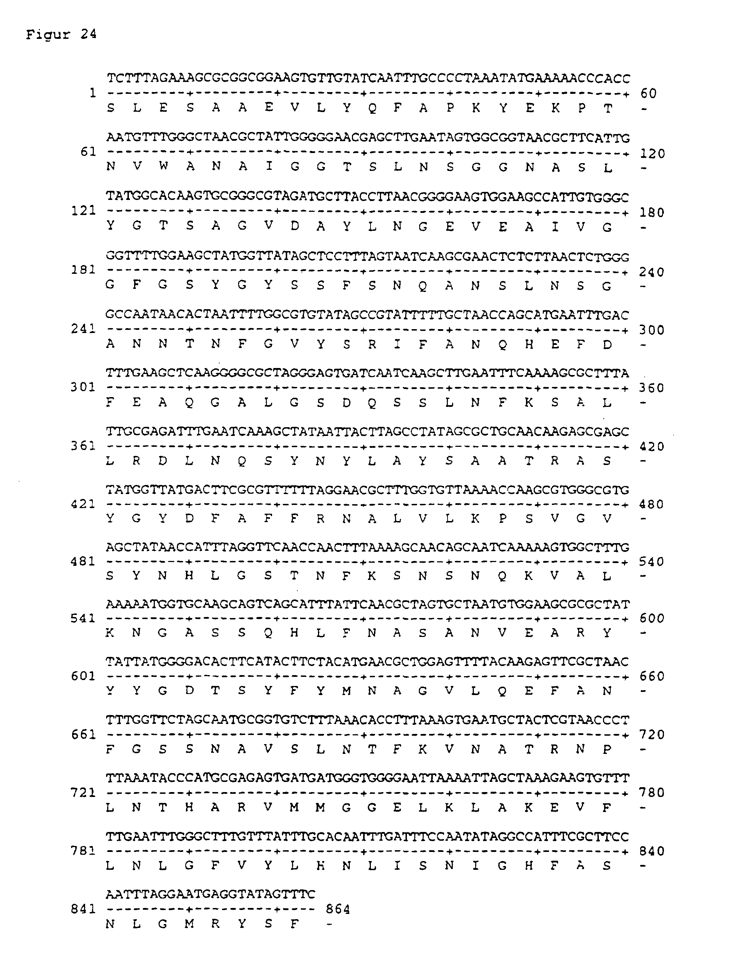

Die DNA-Sequenzen und die davon abgeleiteten Aminosäuresequenzen der zuvor genannten Autotransporter sind in den Figuren 7-24 dargestellt.

Weitere Autotransporterdomänen in bakteriellen Oberflächenproteinen oder in sekretierten bakteriellen Proteinen können aus in Datenbanken vorliegenden Proteinsequenzen,aus in Proteinsequenzen, die auf in Datenbanken verfügbaren DNA-Sequenzen basieren, oder aus durch Sequenzanalyse direkt oder indirekt über die DNA-Sequenz bestimmten Proteinsequenzen abgeleitet werden. Die entsprechenden kodierenden Regionen (Gene) können zur Herstellung von Vektoren oder Fusionsproteingenen, die eine effektive Oberflächenexpression von Passagierproteinen in Gram-negativen Bakterien, insbesondere E.coli ermöglichen, verwendet werden.

Oberflächenpräsentation bzw. -exposition bedeutet erfindungsgemäß, daß die Fusionsproteine bzw. Passagierdomänen auf der dem Medium zugewandten Seite der äußeren Bakterienmembran lokalisiert sind. Oberflächenexponierte Passagierproteine sind in intakten Gram-negativen Bakterien frei zugänglich für Bindungspartner.

In einer bevorzugten Ausführungsform ermöglicht die vorliegende Erfindung also die Oberflächenpräsentation von Peptiden oder in einer weiteren Ausführungsform die Oberflächenpräsentation von Peptidbibliotheken in Gram-negativen Bakterien, insbesondere in E.coli und deren Verwendung zur Bestimmung der Affinität zu einem Antikörper oder einem anderen Rezeptor bzw. zur Epitopkartierung. Epitopkartierung bedeutet, daß das Peptid mit der höchsten Affinität zu einem Antikörper oder einem anderen Rezeptor oberflächenexponiert auf dem produzierenden Stamm identifiziert wird. Dabei wird ein entscheidender Vorteil der vorliegenden Erfindung gegenüber den bisher verwendeten Phagensystemen (Makowski, Gene 128 (1993), 5 - 11) zur Expression von Peptidbibliotheken deutlich. In dem erfindungsgemäßen bakteriellen System erfolgt mit der Identifizierung eines die gewünschten Eigenschaften tragenden Peptides gleichzeitig die Selektion des klonalen Produzenten. Dieser kann unmittelbar vermehrt werden und zur Produktion größerer Mengen des gewünschten Peptides verwendet werden, ohne daß wie im Phagensystem aufwendige Zyklen von Infektion (Phagenvermehrung) und Selektion (Phagenauswahl) notwendig sind. Gleichzeitig mit der Vermehrung des das korrekte Peptid oberflächenexponiert exprimierenden Stammes erfolgt eine Amplifikation des entsprechenden kodierenden Gens, dessen Sequenzanalyse eine eindeutige Identifizierung und Charakterisierung des Peptids mit einfachen und etablierten Techniken erlaubt. Diese erfindungsgemäßen Vorteile treffen auf alle mit der vorliegenden Erfindung oberflächenexponiert exprimierten Passagierdomänen, d.h. Peptide und Polypeptide zu.

Eine erfindungsgemäß hergestellte Peptidbibliothek enthält also Fusionsproteine, zusammengesetzt aus einem Autotransporter, in einer besonders bevorzugten Variante aus dem AIDA Autotransporter, und einem Peptid, das in einem Gram-negativen Bakterium, bevorzugt E.coli oberflächenexponiert produziert wird. Die hohe Varianz an verschiedenen exprimierten Peptiden ergibt sich in einem typischen Beispiel durch die Klonierung von degenerierten, synthetisch hergestellten Oligonucleotiden zwischen die kodierenden Regionen für das Signalpeptid und den Autotransporter.

In einer weiteren bevorzugten Ausführungsform ermöglicht die vorliegende Erfindung die Expression von als Antigen wirkenden Proteinen oder Proteinfragmenten auf der Oberfläche von Gram-negativen Bakterien, bevorzugt von E.coli. Die Konstruktion eines derartigen Fusionsproteines erfolgte erfindungsgemäß unter Verwendung der β-Untereinheit des Toxins von Vibrio cholerae (CtxB) als Passagier und dem AIDA Autotransporter als Trägerprotein. Die Zugänglichkeit der oberflächenexponierten antigenen Domänen für in Frage kommende Bindungspartner wurde erfindungsgemäß nachgewiesen durch Markierung mit einem für CtxB spezifischen Antiserum. Dabei zeigte sich, daß die rekombinanten, in die Außenmembran des E.coli Wirtsstammes eingelagerten Fusionsproteine bis zu 5 % des Gesamtzellproteins ausmachen können, was eine erheblich verbesserte Effizienz im Vergleich zu anderen Systemen bedeutet. Das hier beschriebene Verfahren ermöglicht somit die stabile Produktion und Präsentation von antigen wirkenden Proteinen oder Proteinfragmenten auf der Oberfläche von Gram-negativen Bakterien und in einer bevorzugten Ausführungsform deren Verwendung als Lebendvakzine, zur oralen Vakzinierung oder zum Screening von Seren oder Antikörperbanken. Die Verwendung von Bakterienzellen, beispielsweise attenuierten Salmonella Stämmen (Schorr et al., Vaccine 9 (1991) 675-681), mit oberflächenexponiert exprimierten antigen wirkenden Proteinen hat sich bei der Lebendvakzinierung als vorteilhaft gegenüber der intrazellularen bakteriellen Expression von Antigenen erwiesen.

Generell erlaubt die vorliegende Erfindung in einer bevorzugten Ausführungsform die Oberflächenexpression aller Passagiere, die in ihrem wesentlichen Bestandteil Peptide oder Proteine sind auf der Oberfläche von Gram-negativen Bakterien, insbesondere E.coli.

In einer weiteren bevorzugten Ausführungsform dient die C-terminale Domäne des AIDA Proteins, der AIDA Autotransporter, als Membrananker zur Präsentation rekombinanter Polypeptide des Immunsystems, z.B. rekombinanter Antikörperdomänen auf der Oberfläche von Gram-negativen Bakterien. Die Oberflächenexpression von rekombinanten Antikörperfragmenten ermöglicht deren rasche Modifizierung wie auch die Bewertung und Untersuchung ihrer Antigen-bindenden Eigenschaften. So wird es möglich ganze Bibliotheken von funktionellen Antikörperfragmenten oberflächenexponiert zu produzieren und auf bestimmte vorgegebene Bindungseigenschaften oder Affinitäten hin zu testen. Der Vorteil der vorliegenden Erfindung gegenüber den bisher verwendeten Phagensystemen liegt darin, daB die Variation, d.h. die genetische Manipulation und die Produktion des Proteins in demselben Wirtsorganismus erfolgen kann. Dabei kann die genetische Manipulation eine gezielte sein (ortsspezifische Mutagenese) oder eine zufällige, unter Verwendung von degenerierten Oligonukleotiden zur Synthese einer intakten Fusion aus Antikörper-kodierendem Fragment als Passagier und dem Autotransporter als Trägerprotein. Ebenso kann die genetische Manipulation in Form von in vivo-Mutagenese erfolgen, indem man die Bakterien, die das Gen für das Fusionsprotein enthalten, energiereicher Strahlung (z.B.UV) oder chemischen, mutagen wirkenden Agenzien aussetzt.

Die erfindungsgemäße Selektion des Moleküls mit den korrekten Bindungseigenschaften geht einher mit der Selektion der produzierenden Bakterienzelle. Daraus wird ersichtlich, daß diese erfindungsgemäße Verfahrensweise in ihrer Strategie bestehend aus Variation und nachfolgender Selektion der natürlichen Strategie des Immunsystems zur bestmöglichen Anpassung von Bindungseigenschaften immunogener Moleküle angelehnt ist. Zur Expression funktioneller Antigen-bindender Teile von Antikörpern, die gewöhnlich nicht glykosyliert werden, auf der Oberfläche von Gram-negativen Bakterien, vorzugsweise E.coli sind verschiedene erfindungsgemäße Vorgehensweisen denkbar. Zwei monovalente Fragmente können zusammen präsentiert werden durch getrennte Fusionen der leichten Kette (VL) und der schweren Kette (VH) mit jeweils einer Autotransporterdomäne, die unabhangig voneinander mit verschiedenen kompatiblen Vektoren oder unter Kontrolle verschiedener Promotoren auf dem gleichen Vektor in einer Wirtszelle exprimiert werden. Auf der Oberfläche lagern sich beide oberflächenexponiert vorliegenden Antikörperdomänen zu einem funktionellen Fv-Fragment zusammen, wobei die Stabilität der Interaktion durch eine chemisch induzierte Disulfidbrückenbildung oder anders geartetes chemisches cross-linking gefördert werden kann.

In einer weiteren erfindungsgemäßen Vorgehensweise werden Fusionsproteine hergestellt, die den Autotransporter als Trägerprotein enthalten, und als Passagier die leichte Kette (VL) und die schwere Kette (VH) einer Antigen-bindenden Domäne eines Antikörpers, verknüpft über ein kurzes Linker-Peptid (z.B.[Gly₄Ser]₃), das eine korrekte Zusammenlagerung der beiden Ketten zu einem funktionellen Fv-Fragment erlaubt. Bei der Konstruktion solcher single-chain (sc) Fv-Fragmente ist sowohl eine Verknüpfung des N-Terminus der leichten Kette mit dem C-Terminus der schweren Kette, wie auch eine Verknüpfung des N-Terminus der schweren Kette mit dem C-Terminus der leichten Kette möglich (Pluckthun Immun. Rev. 130 (1992), 151 - 188). Mit den beschriebenen Vorgehensweisen ist auch die Produktion eines kompletten Fab-Fragmentes möglich.

In einer weiteren bevorzugten Ausführungsform ermöglicht die vorliegende Erfindung die oberflächenexponierte Expression von MHC-Klasse II Molekülen in E.coli gegebenenfalls mit definierten eingelagerten Peptiden. Dabei sind zwei Strategien denkbar. In der einen Variante werden zwei verschiedene Fusionsproteine, die beide einen Autotransporter als Trägerprotein enthalten, auf getrennten kompatiblen Vektoren oder auf einem Vektor unter Kontrolle unterschiedlicher Promotoren in einer Wirtszelle exprimiert. Als Passagierprotein dient einmal die α-Kette des gewünschten MHC-Klasse II Subtyps und zum anderen die β-Kette dieses Subtyps, an deren N-Terminus über einen Linker das gewünschte Peptid angehängt sein kann (Kozono et al., Nature 369 (1994) 151-154).

In der zweiten Variante wird ein Passagierprotein bestehend aus dem Peptid, der β-Kette und der α-Kette mit einem Autotransporter fusioniert. Auf der Bakterienoberfläche lagern sich die α- und β-Kette zu einem funktionellen MHC-Molekül zusammen, wobei das Peptid sich korrekt in die Bindungsgrube einlagert. Die Stabilität des Komplexes kann durch chemisch induzierte Disulfidbrückenbildung unterstützt werden. Die Variation des eingelagerten Peptides ist möglich durch ortsspezifische Mutagenese oder/und durch den Einsatz von degenerierten Oligonukleotidprimern bei der Herstellung der die Fusionsproteine kodierenden DNA-Fragmente, ebenso wie durch in vivo-Mutagenese Methoden unter Verwendung von energiereicher Strahlung oder/und chemischen Mutagenen.

Auch hier wird noch einmal der Vorteil des erfindungsgemäßen Verfahrens deutlich. Variation des Bindungspartners, Expression, Selektion des Moleküls mit den optimalen Eigenschaften, Sequenzanalyse und stabile Produktion kann in einem Wirtstamm erfolgen. Damit wird beispielsweise auch eine rasche Charakterisierung von Varianten bereits bekannter Liganden mit besseren Bindungseigenschaften möglich und damit eine Liganden-oder Rezeptoroptimierung.

In einer weiteren bevorzugten Ausführungsform ermöglicht die vorliegende Erfindung die Oberflächenexpression von immunmodulatorischen Rezeptoren wie beispielsweise CD1, Fc-Rezeptor oder MHC-Klasse I Molekülen, sowie deren gezielte Variation.

In weiteren bevorzugten Ausführungsform ermöglicht die vorliegende Erfindung die Oberflächenexpression von T-Zellrezeptoren oder Teilen davon, aber auch von weiteren Oberflächenantigenen eukaryontischer Zellen oder Zellen des Immunsystems.

In einer weiteren bevorzugten Ausführungsform sind die oberflächenexprimierten Proteinfragmente oder Peptide T-Zellepitope, die im Anschluß an die Aufnahme der Bakterien durch adäquate zellinien oder Primärzellen, wie z.B. Makrophagen als in MHC-Moleküle der Klasse I oder II eingelagerte Peptide präsentiert werden und zur Stimulation spezifischer T-Zellen dienen können.

In einer besonders bevorzugten Ausführungsform ermöglicht das erfindungsgemäße Verfahren die Oberflächenexpression und die Variation eines Peptids oder Polypeptids mit einer Affnität zu einem Bindungspartner, eines Liganden, eines Rezeptors, eines Antigens, eines Toxin-bindenden Proteins, eines Proteins mit enzymatischer Aktivität, eines Nukleinsäure-bindenden Proteins, eines Inhibitors, eines Chelator-Eigenschaften habenden Proteins, eines Antikörpers oder einer Antigen-bindenden Domäne eines Antikörpers.

Unter dem Begriff "Bindungspartner" wird erfindungsgemäß ein Element, ein Molekül, eine chemische Verbindung oder ein Makromolekül verstanden, wobei der Bindungspartner und/oder die, die Fusionsproteine exprimierenden Bakterienzellen frei löslich, gebunden an eine Matrix oder aber assoziert mit einer biologischen Membran vorliegen.

Der Begriff "Antigen bindende Domäne" bezeichnet erfindungsgemäß mindestens den Bereich eines Antikörpermoleküls, der hinreichend ist für die spezifische Bindung eines Antigens.

In einer weiteren bevorzugten Ausführungsform ermöglicht die vorliegende Erfindung eine chemische, physikalische oder/und enzymatische Modifikation des oberflächenexponierten Passagierpeptids bzw. -polypeptids oder Teilen davon, wobei die Modifikation eine kovalente Modifikation, eine nicht-kovalente Modifikation, eine Glykosylierung, eine Phosphorylierung oder eine Proteolyse sein kann.

Das erfindungsgemäße Verfahren zur Herstellung einer gegebenenfalls varianten Population von oberflächenexponierten Peptiden und zur Identifizierung von Bakterien, die Peptide bzw. Polypeptide mit einer jeweils gewünschten Eigenschaft tragen, gliedert sich in folgende Schritte:
(1) Herstellen eines oder mehrerer Fusionsgene durch Klonierung der kodierenden Sequenz eines gewünschten Passagiers in frame mit der kodierenden Sequenz der Transporterdomäne des AIDA-Proteins aus E. coli oder einer Variante davon, die eine Homologie von mindestens 80 % zu der Transporterdomäne hat, und eines Signalpeptides, wobei die einzelnen Teilfragmente über PCR amplifiziert oder aus Restriktionsverdauungen anderer DNA stammen können, in mindestens einen Vektor;
(2) gegebenenfalls
   Variieren des Passagiers durch Mutagenese z.B. durch ortspezifische Mutagenese, Verwendung von degenerierten Oligonukleotidprimern in der PCR, durch chemische Mutagenese oder durch Verwendung energiereicher Strahlung;
(3) Einbringen des Vektors oder der Vektoren in Wirtsbakterien;
(4) Exprimieren des Fusionsgens bzw. der Fusionsgene in den Wirtsbakterien, die das Fusionsprotein oder die Fusionsproteine stabil an der Oberfläche präsentieren.
(5) Kultivieren der Bakterien z.B. in Flüssigkultur oder auf Agarplatten zur Produktion des stabil oberflächenexponiert präsentierten Passagiers oder der stabil oberflächenexponiert präsentierten Passagiere;
(6) gegebenenfalls Selektionieren der Bakterien, die den Passagier oder die Passagiere mit den gewünschten Eigenschaften auf der Oberfläche tragen und
(7) gegebenenfalls Charakterisieren eines Bindungspartners für den Passagier mit den optimalen Eigenschaften.

Dabei kann erfindungsgemäß dieses Verfahren mehrmals durchlaufen werden, um die Eigenschaften des oberflächenexponierten Peptids bzw. Polypeptids schrittweise dem gewünschten Bindungsverhalten anzupassen oder in einem ersten Schritt den Bindungspartner bezüglich einer Eigenschaft zu optimieren und in einem zweiten Schritt bezüglich einer oder mehrerer anderer Eigenschaften. Erfindungsgemäß können aber auch in Abhängigkeit der gewünschten Anwendung auch nur einzelne Teilschritte des Verfahrens miteinander verknüpft werden, in ei-nem typische Beispiel die Teilschritte (1), (3), (4) und (5), aber auch alle anderen möglichen Kombinationen.

In einer weiteren bevorzugten Ausführungsform dieses Verfahrens enthält das Fusionsprotein als Trägerprotein den SepA
Autotransporter oder einen Teil davon, oder den *Ics*A
Autotransporter oder einen Teil davon, oder den Tsh
Autotransporter oder einen Teil davon, oder den Ssp
Autotransporter oder einen Teil davon, oder den Hap
Autotransporter oder einen Teil davon, oder den Prn
Autotransporter oder einen Teil davon, oder den Hsr
Autotransporter oder einen Teil davon, oder den BrkA
Autotransporter oder einen Teil davon, oder den VacA
Autotransporter oder einen Teil davon oder einen Rickettsia-
Autotransporter oder einen Teil davon,
die jeweils die Sezernierung des Fusionsproteines ermöglichen.

Die Expression multimerer Proteine wird erfindungsgemäß möglich durch Herstellung verschiedener Fusionsproteine in einer Zelle, die sich auf der Oberfläche zu einer funktionellen Einheit zusammenlagern.

Die geringe Generationszeit der als Wirtsorganismen verwendbaren Bakterien ermöglicht einen permanenten Zyklus von Variation und Selektion, der eine evolutionsartige Anpassung des Passagierproteins , aber auch des Autotransporters an vorgegebene Eigenschaften ermöglicht. Dabei kann es sich in einem typischen Beispiel um Bindungsaffinitäten zwischen dem Passagierprotein und einem Bindungspartner handeln. Die Isolierung der Bakterien mit dem stabil exponierten Fusionsprotein erfolgt in einer bevorzugten Ausführungsform dieses Verfahrens durch Bindung an einen immobilisierten oder/und markierten Bindungspartner, z.B. einen Matrix-fixierten Bindungspartner, an einen Fluoreszenz-markierten Bindungspartner, einen Magnetpartikel-markierten Bindungspartner oder einen chromogen markierten Bindungspartner. In einer weiteren bevorzugten Ausführungsform ist der Bindungspartner so modifiziert, daß er in einem zweiten Schritt durch einen für die Modifikation spezifischen Bindungspartner detektiert werden kann.

Ein weiteres Ziel der vorliegenden Erfindung ist die Bereitstellung von stabil exprimierten Fusionsproteinen oder Teilen davon oder von Bakterien mit stabil auf der Oberfläche exprimierten Fusionsproteinen und deren Verwendung für therapeutische Zwecke oder diagnostische Zwecke, bei der Schadstoffanreicherung oder Entfernung, bei der Inaktivierung von Toxinen, bei der Rohstoffmobilisierung, bei der Lebensmittelherstellung oder -verarbeitung, bei der Waschmittelherstellung, bei der Markierung von ausgesuchten eukaryontischen oder prokaryontischen Zellen. Erfindungsgemäß können Antikörper oder Antikörperfragmente stabil auf der Oberfläche exprimierende Bakterien, in einem typischen Beispiel unter Verwendung des AIDA Autotransporters als Transporterdomäne zur Produktion derselben verwendet werden, wobei diese Antikörper oder Antikörperfragmente anschließend, gegebenenfalls nach einer Aufreinigung für diagnostische oder therapeutische Zwecke eingesetzt werden. Mit solchen Antikörpern oder Antikörperfragmenten wäre es beispielsweise möglich, Zellen mit bestimmten Oberflächenmarkern, als typisches Beispiel seien hier Tumorantigene genannt, spezifisch zu bezeichnen oder zu selektionieren. In einem weiteren typischen Beispiel handelt es sich bei den markierten Oberflächenmarkern um Rezeptoren, wobei die Markierung einhergeht mit der Blockierung der oder einer der Rezeptoreigenschaften, womit eine gezielte Inhibition einer durch den Rezeptor ausgelösten oder vermittelten Signaltransduktion und der damit verbundenen Zellfunktion möglich wird.

### Beschreibung der Figuren

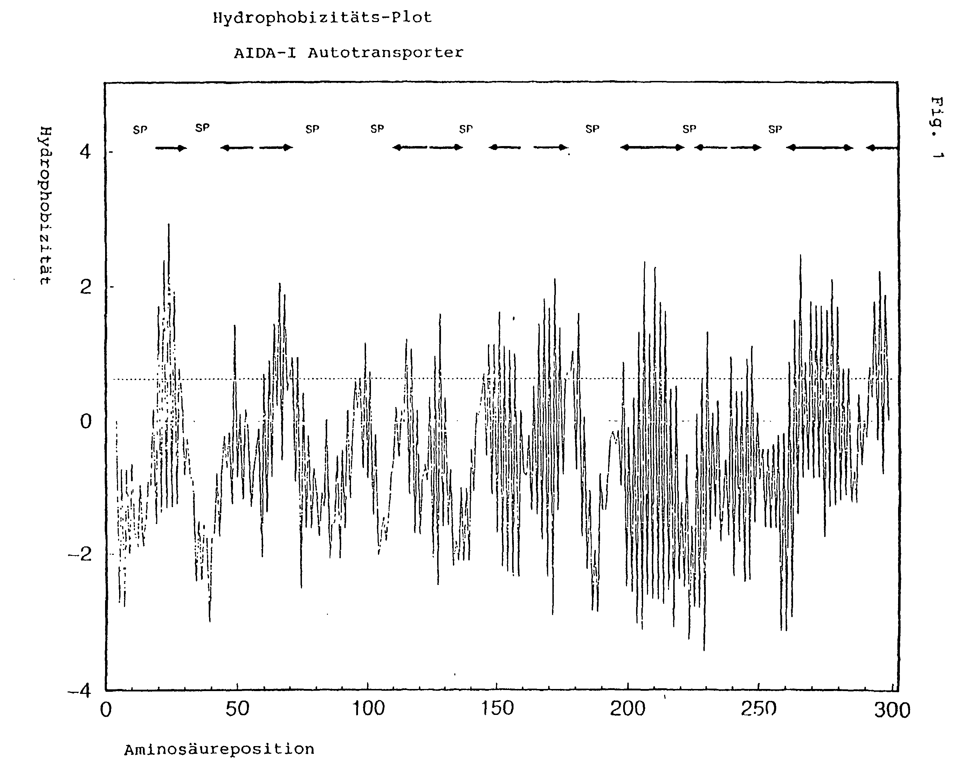

Figur 1:
Hydrophobizität der C-terminalen 300 Aminsoäuren des AIDA-I Proteins.
Die für Autotransporter typische Pore in der Außenmembran Gram-negativer Bakterien wird gebildet durch amphipathische β-Faltblattstrukturen, d.h. von Domänen mit β-Faltblattstruktur und alternierend hydrophoben und hydrophilen Aminosäuren. Dies kann man sichtbar machen, indem man einen relativen Hydrophobizitätswert der Aminosäure, der mittels eines bestimmten Algorithmus der Aminosäure zugeordnet wurde, gegen die Position der Aminosäure aufträgt. Es wurde der Algorithmus von Vogel und Jähnig (J. MoL Biol. 190 (1986) 191-199) verwendet. Die Pfeile zeigen die möglichen Membrandurchgänge an, wobei Pfeil nach links bedeutet, daß der Membrandurchgang von innen nach außen verläuft und Pfeil nach rechts Membrandurchgang von außen nach innen anzeigt. SP deutet eine relative Oberflächenwahrscheinlichkeit der Aminosäuren berechnet nach Emini et al. (J. Virol. 55 (1985), 836-839) an.
Figur 2:
Modell des Autotransporters aus dem AIDA-I Protein.
Ausgehend von der Auftragung der relativen Hydrophobizität einer Aminosäure gegen ihre Position (Figur 1) läßt sich die durch die antiparallelen, amphipathischen β-Faltblätter gebildete Faßstruktur als Modell darstellen. Die hier dargestellte aufgeschnittene Faßstruktur, wird in der Membran durch Interaktion des ersten mit dem antiparallelen letzten Membrandurchgang geschlossen. Die in Rauten geschriebenen Aminosäuren befinden sich im Membranbereich, wobei fett umrandete relativ hydrophob sind und zur Außenseite des Faßes, also zur Membran hin orientiert sind, während dünn umrandete relativ hydrophil sind und mit ihren Seitenketten zur Innenseite der Pore hin zeigen. In Kreisen gezeichnete Aminosäuren bilden Schleifen außerhalb der Membran. Alanin an Position 1 des Modells trägt in der vollständigen Sequenz von AIDA-I die Nummer 1014, während das terminale Phenylalanin in der vollständigen Sequenz die Nummer 1286 trägt (Benz und Schmidt, Mol Microbiol 11 (1992),1539-1546).
Figur 3 a:
Herstellung von pJM7, eines Vektors zur Oberflächenexpression von CtxB.
pJM7 enthält eine Genfusion (FP59) aus dem Choleratoxin B und der AIDA-Linker-/β- Faß-Region. Diese Genfusion wird unter der Kontrolle des künstlichen Promotors PTK (Klauser et al, EMBO J. 9 (1990) 1991-1999) in einem Vektor mit hoher Kopienzahl konstitutiv exprimiert. Das ctxB-Gen wurde mit den Oligonukleotiden EF16 und JM6 aus dem Plasmid pTK1 (Klauser et al, EMBO J. 9 (1990) 1991-1999) durch PCR amplifiziert. Der Autotransporter, bestehend aus dem β-Faß und der Linkerregion aus AIDA-I wurde durch Amplifikation mit den Oligonukleotiden JM1 und JM7 aus einer Plasmid-DNA-Präparation von E.coli EPEC 2787 (Benz und Schmidt, Infect. Immun. 57 (1989), 1506-1511) amplifiziert. Das Oligonukleotid JM 1 enthält in seinem 5'-Überhang eine BglII-Erkennungssequenz, die Oligonukleotide JM6 und JM7 enthalten je eine KpnI-Erkennungssequenz. Die Vektor-DNA (pBA) wurde mit ClaI und BamHI hydrolysiert und die beiden PCR-Produkte wurden im Anschluß an die Amplifikation mit ClaI und KpnI (EF16/JM6-Fragment) bzw. mit BglII und KpnI (JM7/JM1-Fragment) nachgeschnitten. Die so generierten drei Fragmente wurden in einer Ligation kondensiert.
Figur 3 b:
Herstellung von pJM22, eines Vektors zur Oberflächenexpression von Peptiden.
pJM22 produziert das Fusionsprotein FP50, das aus drei Domänen besteht. Am N- terminalen Ende liegt die CtxB-Signalsequenz, die für den Export des entstehenden Fusionsproteins über die Zellmembran (Sec-vermittelt) sorgt. Daran anschließend folgt die Passagierdomäne, in diesem Fall ein Peptid, das Epitop PEYFK. C-terminal endet das Fusionsprotein mit der AIDA-β-Faß/Linker-Region, dem Autotransporter, welcher die N-terminal um das Signalpeptid verkürzte Passagierdomäne auf die Oberfläche von E.coli befördert. Zur Konstruktion von pJM22 wurde zunächst die DNA von pJM7 mit XhoI hydrolysiert und der Vektoranteil von pJM7 durch PCR mit den Oligonukleotiden JM7 und JM20 amplifiziert. Dabei wurde das ctxB-Gen mit Ausnahme seiner Signalsequenz deletiert. Das Oligonukleotid JM20 enthielt in seinem 5'-Überhang zusätzlich zu der KpnI-Schnittsequenz fünf Codons, die für die Aminosäuren PEYFK kodieren. Diese Aminosäurenabfolge stellt ein lineares Epitop für den monoklonalen Antikörper Dü142 dar. Das PCR-Produkt wurde mit KpnI hydrolysiert und anschließend mit sich selbst ligiert.
Figur 4
Expressionsnachweis und Proteasesensitivität.
Aufgrund der starken stabilen Expression der Fusionsproteine FP59 (von pJM7 aus) und FP50 (von pJM22 aus) in E.coli sind diese leicht in einem mit Coomassie Brilliant Blue gefärbten Ganzzellysat zu identifizieren. Proteasezugänglichkeit stellt ein übliches Mittel zur Bestimmung der Lokalisierung eines Proteins dar. Zu zelleigenen Proteinen ist nur dann Zugang zu erwarten, wenn diese auf der Außenseite der Bakterie präsentiert werden oder wenn die Außenmembran der Bakterie für Proteasen durchlässig wird. Um letzteres auszuschließen kann man einen Protease-sensitiven Marker benutzen, von dem bekannt ist, daß er natürlicherweise im Periplasma vorliegt. Nur wenn dieser nicht von der eingesetzten Protease angegriffen wird, ist die Integrität der Außenmembran gewährleistet. Zellen von E.coli UT5600 bzw. JK321 wurden über Nacht auf LB-Agar (50 mg/l Ampicillin) angezogen und in PBS suspendiert. Die Zellsuspensionen wurde auf eine OD578 = 4,0 eingestellt. Zellen von 0,5 ml Zellsuspension wurden 1 min in der Tischzentrifuge sedimentiert und in 200 µl PBS mit 0,1 mg/ml Protease resuspendiert. Die Ansätze wurden 20 min bei 37° C inkubiert und durch Abkühlung auf 0° C, einminütiges Sedimentieren und Resuspendieren des Pellets in 40 µl SDS-PAGE-Probenpuffer und sofortiges fünfzehnminütiges Kochen gestoppt. Die Auswertung erfolgte nach SDS-PAGE durch Western-Blotting (4b und 4c) oder durch Färbung mit Coomassie Brilliant Blue (4a). Um auszuschließen, daß die Proteasen Zugang zum Periplasma hatten, wurden nicht nur Antiseren, die spezifisch für die Passagierproteindomänen sind, eingesetzt sondern auch ein Antiserum spezifisch für den C-terminalen Teil von OmpA, der natürlicherweise unzugänglich im Periplasma vorliegt und deshalb durch extern zugesetzte Proteasen wie Trypsin nicht angreifbar sein sollte (4c).
Figur 4a:
SDS-PAGE und anschließende Färbung mit Coomassie Brilliant Blue zum Nachweis der Proteasesensitivität und zur Quanhfizierung der Expression. Aufgetragen wurden Ganzzellysate von E.coli JK321 und E.coli UT5600.

| | |
|---|---|
| Spur 1 | JK321 pJM7 C * |
| Spur 2 | JK321 pJM7 T** |
| Spur 3 | JK321 pJM7 -*** |
| Spur 4 | Molekulargewichtsmarker (94, 67, 43, 30, 20 und 14 kDa) |
| Spur 5 | JK321 pJM22 C |
| Spur 6 | JK321 pJM22 T |
| Spur 7 | JK321 pJM22 - |
| Spur 8 | JK321 pTK61 C |
| Spur 9 | JK321 pTK61 T |
| Spur 10 | JK321 pTK61 - |
| Spur 11 | UT5600 pJM7 C |
| Spur 12 | UT5600 pJM7 T |
| Spur 13 | UT5600 pJM7 - |
| Spur 14 | Molekulargewichtsmarker (94, 67, 43, 30, 20 und 14 kDa) |
| Spur 15 | UT5600 pJM22 C |
| Spur 16 | UT5600 pJM22 T |
| Spur 17 | UT5600 pJM22 - |
| Spur 18 | UT5600 pTK61 C |
| Spur 19 | UT5600 pTK61 T |
| Spur 20 | UT5600 pTK61 - |

| | |
|---|---|
| C* Zellen wurden mit Chymotrypsin verdaut | |
| T** Zellen wurden mit Trypsin verdaut | |
| *** native Zellen | |

Figur 4 b:
Western-Blot zum Nachweis der Expression und der Proteasesensitivität
Aufgetragen wurden Ganzzelllysate von E.coli JK321 und E.coli UT5600. Nach der Elektrophorese wurden die Proteine aus dem Gel nach dem Semi-dry-Verfahren auf eine Nitrozellulosemembran übertragen. Anschließend wurden die Filter mit Blocklösung (PBS mit 0,5% Tween 20 und 0,5 M NaCl) 10 min blockiert und das erste Antiserum, AK55 (Kaninchen-Anti-Choleratoxin B) 1:200 in Blocklösung verdünnt, zugegeben. Zum Nachweis des Epitops PEYFK wurde der Hybridom-Überstand Dü142 1: 35 verdünnt in Blocklösung zugegeben. Die Filter wurden 1 h mit den primären Antikörpern inkubiert, anschließend dreimal gewaschen und 30 min mit ProteinA-Alkalische-Phosphatase-Konjugat (1: 500 in Blocklösung) inkubiert. Die Filter wurden mit NBT/BCIP-Färbelösung entwickelt.

| | |
|---|---|
| Spur 1 | JK321 pJM7C * |
| Spur 2 | JK321 pJM7 T** |
| Spur 3 | JK321 pJM7 -*** |
| Spur 4 | Molekulargewichtsmarker (106, 80, 50, 32, 27 und |
| | 18 kDa) |
| Spur 5 | JK321 pJM22 C |
| Spur 6 | JK321 pJM22 T |
| Spur 7 | JK321 pJM22 - |
| Spur 8 | JK321 pTK61 C |
| Spur 9 | JK321 pTK61 T |
| Spur 10 | JK321 pTK61 - |
| Spur 11 | UT5600 pJM7 C |
| Spur 12 | UT5600 pJM7 T |
| Spur 13 | UT5600 pJM7 - |
| Spur 14 | Molekulargewichtsmarker (106, 80, 50, 32, 27 und 18 kDa) |
| Spur 15 | UT5600 pJM22 C |
| Spur 16 | UT5600 pJM22 T |
| Spur 17 | UT5600 pJM22 - |
| Spur 18 | UT5600 pTK61 C |
| Spur 19 | UT5600 pTK61 T |
| Spur 20 | UT5600 pTK61 - |

| | |
|---|---|
| C* Zellen wurden mit Chymotrypsin verdaut | |
| T** Zellen wurden mit Trypsin verdaut | |
| -*** native Zellen | |

Figur 4 c:
Nachweis der Integrität der Außenmembran durch Western-Blot-Analyse.
Aufgetragen wurden Ganzzellysate von E.coli JK321 und E.coli UT5600. Nach der Elektrophorese wurden die Proteine aus dem Gel nach dem Semi-dry-Verfahren auf eine Nitrozellulosemembran übertragen. Anschließend wurden die Filter mit Blocklösung (PBS mit 0,5% Tween 20 und 0,5 M NaCl) 10 min blockiert und das erste Antiserum, K56 (Kaninchen Anti-OmpA) 1: 1000 in Blocklösung verdünnt, zugegeben. Die Filter wurden 1 h mit dem primären Antiserum inkubiert, anschließend dreimal gewaschen und 30 min mit Protein A Alkalische-Phosphatase-Konjugat (1: 500 in Blocklösung) inkubiert. Entwickelt wurden die Filter mit NBT/BCIP-Färbelösung. OmpA ist ein Außenmembranprotein von E.coli mit einem C-terminalen periplasmatischen Anteil. Dieser periplasmatische Teil ist Trypsin-sensitiv. Wenn Trypsin Zugang zum Periplasma hat, wird vom reifen OmpA (35 kDa) ein circa 10 - 11 kDa großer Teil abgedaut. Ein Verdau würde also in einer Versetzung der OmpA-Bande im Western-Blot von 35 kDa nach 25 kDa resultieren (Klauser et al, EMBO J. 9 (1990) 1991- 1999), was bei Verwendung des AI-DA-I Autotransporters zum Transport rekombinanter Proteine offensichtlich nicht der Fall ist

| | |
|---|---|
| Spur 1 | JK321 pTK1 T* |
| Spur 2 | JK321 pJM7 T |
| Spur 3 | JK321 pJM22 T |
| Spur 4 | JK321 pTK61 T |
| Spur 5 | Molekulargewichtsmarker (106, 80, 50, 32, 27 und 18kDa) |
| Spur 6 | JK321 pTK1 -** |
| Spur 7 | JK321 pJM7 - |
| Spur 8 | JK321 pJM22 - |
| Spur 9 | JK321 pTK61 - |
| Spur 10 | leer |
| Spur 11 | UT5600 pTK1 T* |
| Spur 12 | UT5600 pJM7 T |
| Spur 13 | UT5600 pJM22 T |
| Spur 14 | UT5600 pTK61 T |
| Spur 15 | Molekulargewichtsmarker (106, 80, 50, 32, 27 und 18kDa) |
| Spur 16 | UT5600 pTK1 -** |
| Spur 17 | UT5600 pJM7 - |
| Spur 18 | UT5600 pJM22 - |
| Spur 19 | UT5600 pTK61 - |

| | |
|---|---|
| T* Zellen wurden mit Trypsin verdaut | |
| ** native Zellen | |

Figur 5
Immunfluoreszenz
Die Immunfluoreszenz ganzer, nicht permeabilisierter Zellen stellt eine übliche Methode des Nachweises auf der Zelloberfläche exponierter Determinanten dar. Die dabei zur Detektion der Determinanten eingesetzten Antikörper sind zu groß, um durch die intakte Außenmembran zu gelangen. Zur Unterscheidung und zur Abschätzung der Hintergrundaktivität von periplasmatisch oder zellulär exprimierten Determinanten benutzt man als Kontrolle Antikörper gegen bekanntermaßen periplasmatisch bzw. zellulär exprimierte Antigene.
Zellen von E.coli UT5600, die eines der Plasmide pBA, pTK1, pTK61, pJM7 oder pJM22 enthielten, wurden über Nacht auf LB-Agar (Ampicillin 50 mg/l) angezogen und in PBS bis zu einer optischen Dichte von 0,1 bei 578 nm suspendiert. Mit 500 µl dieser Zellsuspension wurden Deckgläser, die in 24 Loch-Mikrotiterplatten lagen, überschichtet. Die Zellen wurden 5 min in der Plattenzentrifuge auf die Deckgläser sedimentiert. 450 µl des Überstands wurden abgehoben und durch PBS mit 2,5% PFA (Paraformaldehyd) ersetzt, womit 20 min lang fixiert wurde. Der Überstand wurde gänzlich abgehoben und es wurde dreimal mit 500 µl PBS gewaschen. Unspezifische Bindungsstellen wurden durch 5 min Inkubation mit 300 µl PBS, das 1% FCS enthielt, blockiert. Die Blockierungslösung wurde restlos abgehoben, die Deckgläser in ihren Vertiefungen zentriert, mit 15 µl einer 1:100 Verdünnung des Kaninchenserums AK55 (gegen Choleratoxin B entwickelt) bedeckt und 1 h bei Raumtemperatur in einer feuchten Kammer inkubiert. Anschließend wurde dreimal mit je 500 µl PBS gewaschen, 5 min mit 350 µl PBS/FCS blockiert und mit 15 µl einer 1: 100 Verdünnung eines Ziege--Anti-Kaninchen-Texasrot-Konjugats 30 min inkubiert. Nach folgendem dreimaligem Waschen wurden die Deckgläser auf Objektträger gelegt und mit Einbettungsmedium eingebettet. Das Ergebnis der Immunfluoreszenz wurde mikroskopisch beurteilt und bei gleichlangen Belichtungszeiten fotographisch festgehalten.
a) E.coli UT5600 pBA (als Negativkontrolle benutzter Stamm, der nur den Klonierungsvektor ohne Insert enthält)
b) E.coli UT5600 pTK1 (produziert das Choleratoxin B, das ins Periplasma exportiert wird. Dieses Konstrukt dient zur Bestimmung der Hintergrundaktivität des periplasrnatisch exprimierten Choleratoxin B).
c) E.coli UT5600 pJM7 (exprimiert FP59, das Fusionsprotein aus AIDA und Choleratoxin B, welches auf der Oberfläche von E.coli präsentiert wird).
d) E.coli UT5600 pJM22 (exprimiert FP50, das Fusionsprotein aus AIDA und dem Epitop PEYFK. Mit diesem Konstrukt wird demonstriert, daß der AIDA-Anteil der FP59 und FP50 keine Kreuzreaktivität mit dem in diesem Experiment benutzten AK55 aufweist).
e) E.coli UT5600 pTK61 (produziert ein Fusionsprotein aus Choleratoxin B und Iga-β, das auf der Oberfläche von E.coli präsentiert wird (Klauser et al., EMBO J. 9 (1990) 1991- 1999). Dient dem Vergleich mit dem AIDA-Konstrukt FP59).

Figur 6
DNA-Sequenzen der verwendeten Oligonucleotide
Figuren 7-24
DNA-Sequenz (nicht-kodierender Strang) und davon abgeleitete Aminosäuresequenzen von bakteriellen Autotransportern.
Figur 7
Darstellung des in die Membran integrierten Teils des Autotransporters von AIDA-I aus Escherichia coli (Benz und Schmidt, Mol.Microbiol. 6 (1992), 1539-1546).
Figur 8
Darstellung des in die Membran integrierten Teils des Autotransporters von BrkA aus Bordetella pertussis (Fernandez und Weiss, Infect Immun. 62 (1994), 4727-4738).
Figur 9
Darstellung des in die Membran integrierten Teils des Autotransporters von Hap aus Haemophilus influenzae (StGeme et al., Mol.Microbiol. 14 (1994), 217-233).
Figur 10
Darstellung des in die Membran integrierten Teils des Autotransporters von Hsr aus Helicobacter mustelae (O'Toole et al., Mol.Microbiol 11 (1994), 349-361).
Figur 11
Darstellungen des in die Membran integrierten Teils des Autotransporters von IcsA aus Shigella flexneri (Goldberg et al., J.Bacteriol 175 (1993), 2189-2196).
Figur 12
Darstellung des in die Membran integrierten Teils des Autotransporters von Prn (outer membrane protein P96) aus Bordetella pertussis (Charles et al., Proc.Natl.Acad.Sci. USA 86 (1989), 3554-3558).
Figur 13
Darstellung des in die Membran integrierten Teils des Autotransporters von Prn (P70,Pertactin) aus Bordetella parapertussis (Li et al., J.Gen.Microbiol. 138 (1992), 1697-1705).
Figur 14
Darstellung des in die Membran integrierten Teils des Autotransporters von dem 190 kDa cell surface antigen aus Rickettsia rickettsii (Anderson et al., unveröffentlicht, Genbank-Accessionnr. M31227).
Figur 15
Darstellung des in die Membran integrierten Teils des Autotransporters von SpaP aus Rickettsia prowazekii (Carl et al., Proc.Natl.Acad.Sci. USA 87 (1990), 8237-8241).
Figur 16
Darstellung des in die Membran integrierten Teils des Autotransporters aus dem 120 kilodalton outer membrane protein (rOmp B) von Rickettsia rickettsii (Gilmore et al., Mol.Microbiol. 5 (1991), 2361-2370).
Figur 17
Darstellung des in die Membran integrierten Teils des Autotransporters von SlpT aus Rickettsia typhi (Hahn et al., Gene 133 (1993), 129-133).
Figur 18
Darstellung des in die Membran integrierten Teils des Autotransporters von SepA aus Shigella flexneri (Benjellou-Touimi et al., Mol.Microbiol 17 (1995), 123-135).
Figur 19
Darstellung des in die Membran integrierten Teils des Autotransporters von Ssp aus Serratia marcescens RH1 (Rho, unveröffentlicht, Genbank-Accessionnr. X59719).
Figur 20
Darstellung des in die Membran integrierten Teils des Autotransporters von Ssp von S.marcescens IFO-3046, clone pSP11 (Yanagida et al., J.Bacteriol. 166 (1986), 937-944).
Figur 21
Darstellung des in die Membran integrierten Teils des Autotransporters von Ssp-h1 aus Serratia marcescens, strain IFO3046 (Onishi und Horinouchi, unveröffentlicht, Genbank-Accessionnr. D78380).
Figur 22
Darstellung des in die Membran integrierten Teils des Autotransporters von Ssp-h2 aus Serratia marcescens, strain IFO3046 (Onishi und Horinouchi, unveröffentlicht, Genbank-Accessionnr. D78380).
Figur 23
Darstellung des in die Membran integrierten Teils des Autotransporters von Tsh aus Escherichia coli (Provence et al. 1994, Infect. Immun. 62 (1994), 1369-1380).
Figur 24
Darstellung des in die Membran integrierten Teils des Autotransporters von VacA aus Helicobacter pylori (Schmitt und Haas, Mol.Microbiol. 12 (1994), 307-319). Von VacA sind noch mindestens 3 weitere Formen bei Helicobacter pylori bekannt, die sich aber in dem angegebenen Bereich nicht wesentlich unterscheiden.

### BEISPIELE

### Beispiel 1:

Identifizierung und Lokalisierung des Autotransporters in einem Oberflächenprotein von Escherichia coli.

Um einen für die gewünschte Anwendung adäquaten Autotransporter, d.h. angepaßt an das Passagierprotein und den zu verwendenden Wirtsstamm, zu finden, ist es notwendig eine Analyse der C-terminalen Aminosäuresequenz eines in Frage kommenden Proteins durchzuführen. Dabei kann es sich um ein bereits als Oberflächenfaktor identifiziertes Protein handeln, aber auch um eine in einer Datenbank abgelegte Aminosäuresequenz eines Proteins mit unbekannter Funktion, um eine von einer in einer Datenbank abgelegten DNA-Sequenz abgeleitete Aminosäuresequenz eines Proteins oder die im Anschluß an eine Sequenzanalyse von einem Gen abgeleitete Aminosäuresequenz eines Proteins. Der N-Terminus des Proteins sollte eine Signalpeptidsequenz enthalten um einen Transport über die innere Membran zu ermöglichen und am C-Terminus sollte der in die Membran intergrierte Teil mit der aromatischen Aminosäure Phenylalanin oder Tryptophan beginnen, gefolgt von abwechselnd polaren (oder geladenen) und hydrophoben (oder aromatischen) Aminosäuren. Die Passagierdomäne sollte wenig Cysteine und überhaupt keine Disulfidbrücken enthalten, da sich gezeigt hat, daß diese einen Transport des Passagiers durch die gebildete Pore blockiert. Der Hydrophobizitätsplot sollte eine gerade Anzahl von amphipathischen β-Faltblattstrukturen anzeigen, aus denen sich die Außenmembranpore konstituiert. Die amphipathischen β-Faltblattstrukturen sollten ca. 12 Aminosäuren lang sein und ein Minimum an geladenen Aminosäuren zur Membranseite hin orientiert enthalten, wobei die die Membrandurchgänge verbindenden Schleifen zum Periplasma hin wenige Aminosäuren enthalten. Zur Außenseite (Medium) hin können erheblich mehr Aminosäuren vorliegen. Daraus ergibt sich im Hydrophobizitätsplot eine Zusammenlagerung der Membrandurchgänge in antiparallelen Paaren mit Ausnahme des ersten und des letzten Membrandurchgangs, die durch antiparallele Zusammenlagerung miteinander die Faßstruktur der Pore vollenden. Ausgehend von der Erfüllung dieser Kriterien läßt sich nun ein Modell des Autotransporters aufstellen, mit Hilfe dessen die Lage und Ausdehnung der für den Transport notwendigen Aminosäuren festgelegt werden kann. Zusätzlich zu den für die Pore notwendigen Aminosäuren muß für einen funktionsfähigen Autotransporter auch noch eine sogenannte Linkerregion, die vom periplasmatisch gelegenen N-Terminus der β-Faßstruktur durch die Pore an die Oberfläche verläuft, in das Fusionsprotein mitübernommen werden, damit die Oberflächenexposition aller Passagierdomänen vollständig gewährleistet ist.

Erstes Ziel der vorliegenden Erfindung war es ein System zur optimierten Oberflächenexposition rekombinanter Proteine in E.coli bereitzustellen. Deshalb wurde nach einem Autotransporter in einem natürlichen Oberflächenprotein von E.coli gesucht. Die Wahl fiel auf das Adhesin AIDA-I (Adhesin Involved in Diffuse Adherence, Benz und Schmidt Infect. Immun. 57 (1989) 1506-1511), dessen Sequenz in Datenbanken verfügbar war. Erfindungsgemäß zeigte sich eine Signalsequenz am N-Terminus von 49 Aminosäuren, während am C-Terminus die erfindungsgemäßen Vorgaben durch die Aminosärenabfolge FSYKI (Phenylalanin-Serin-Tyrosin-Lysin-Isoleucin) erfüllt waren. Die transportierte Domäne enthielt keine Cysteine und durch den Hydrophobizitätsplot (Figur 1) wurden 14 antiparallele, amphipathische β- Faltblattstrukturen vorhergesagt. Somit sind für die Bildung der Pore mindesten die Aminosäuren von Alanin an Position 1014 der gesarnten Aminosäuresequenz (Benz und Schmidt, Mol. Microbiol. 6 (1992) 1539-1546) bis zu Phenylalanin an Position 1286 notwendig (Figur 2). Als Linkerregion wurden zusätzlich Aminosäuren, die sich N-terminal an das Alanin 1014 anschließen ausgewählt. Die somit ausgewählte funktionelle Autotransporterregion konnte nun mittels PCR aus der DNA des entsprechenden E.coli EPEC2787 isoliert werden und zur Konstruktion eines Fusionsproteins verwendet werden.

### Beispiel 2:

### Konstruktion eines oberflächenexponierten Fusionsproteins mit einer antigenen Determinante als Passagierprotein

Ausgehend von den Annahmen, daß es sich bei AIDA-I um einen Autotransporter handelt und daß eine Genfusion aus einem beliebigen Passagier und einem E.coli-eigenen Autotransporter (nämlich AIDA-β) für E.coli besser verträglich sein sollte als eine Genfusion desselben Passagiers mit einem heterologen Autotransporter (z.B. Iga-β) wurde eine Genfusion zwischen aida-β und einem Gen für ein Passagierprotein vorgenommen. Um den Transport des Passagiers zu gewährleisten, wurde nicht nur AIDA-β sondern auch eine N-terminal vom β-Faß gelegene Verbindungsregion ("Linker") kloniert.

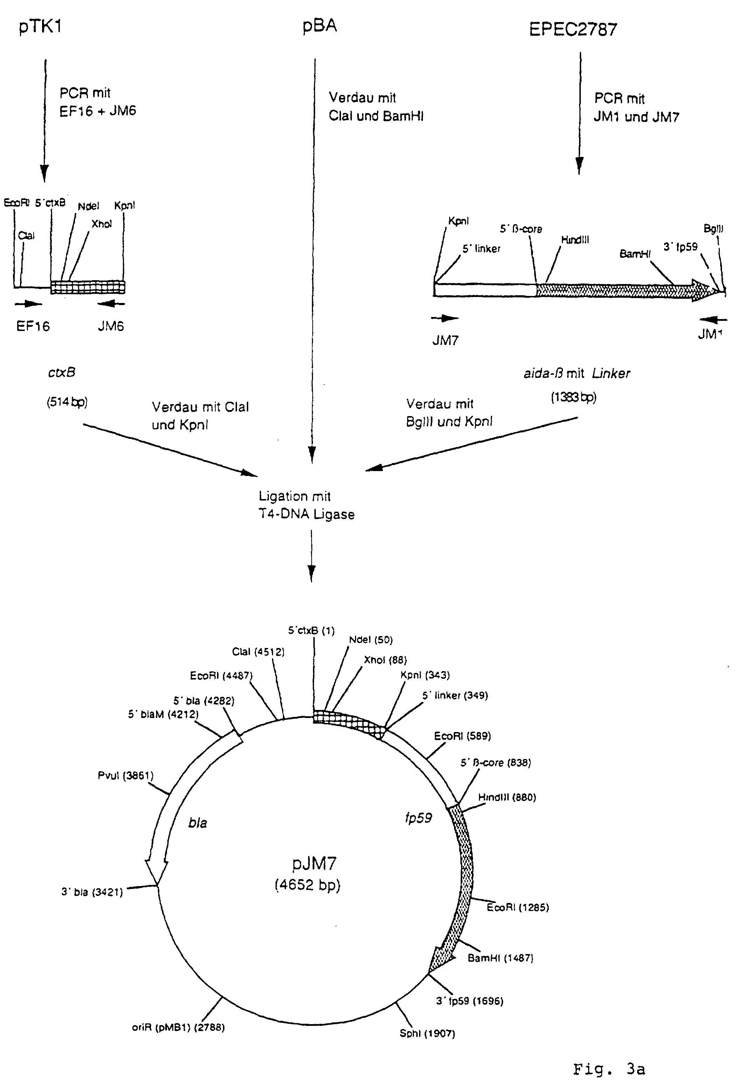

Als Passagier wurde CtxB ausgewählt und das entsprechende Gen aus pTK1 (Klauser et al, EMBO J. 9 (1990), 1991-1999) mit den Oligonukleotiden EF16 und JM6 mittels PCR amplifiziert. Da AIDA-I Plasmid-kodiert in E.coli EPEC 2787 (Benz und Schmidt, Infect. Immun. 57 (1989), 1506-1511) vorliegt, wurde der AI-DA-I Autotransporter mit Linkerregion ebenfalls durch PCR mit den Oligonukleotiden JM1 und JM7 aus einer Plasmidpräparation von E.coli EPEC 2787 amplifiziert. Die beiden PCR-Produkte wurden mit Restriktionsenzymen verdaut, deren Erkennungssequenzen in den Oligonukleotiden enthalten waren. Die beiden so entstandenen Fragmente wurden in einen passend vorverdauten Klonierungsvektor (pBA) mit hoher Kopienzahl kloniert. So entstand ein Konstrukt mit einem künstlichen konstitutiven Promotor (PTK; Klauser et al., EMBO J. 9 (1990) 1991-1999) vor einer Genfusion bestehend aus ctxB am 5'-Ende (kodierend für die Aminosäuren 1-113), gefolgt von einem AIDA-I Linker (kodierend für die Aminosäuren 116-279 des Fusionsproteines) und dem AIDA-I Autotransporter (kodierend für die Aminosäuren 280-563 des Fusionsproteines) am 3'-Ende (Figur 3a). Die entstandene Genfusion wurde FP59 benannt.

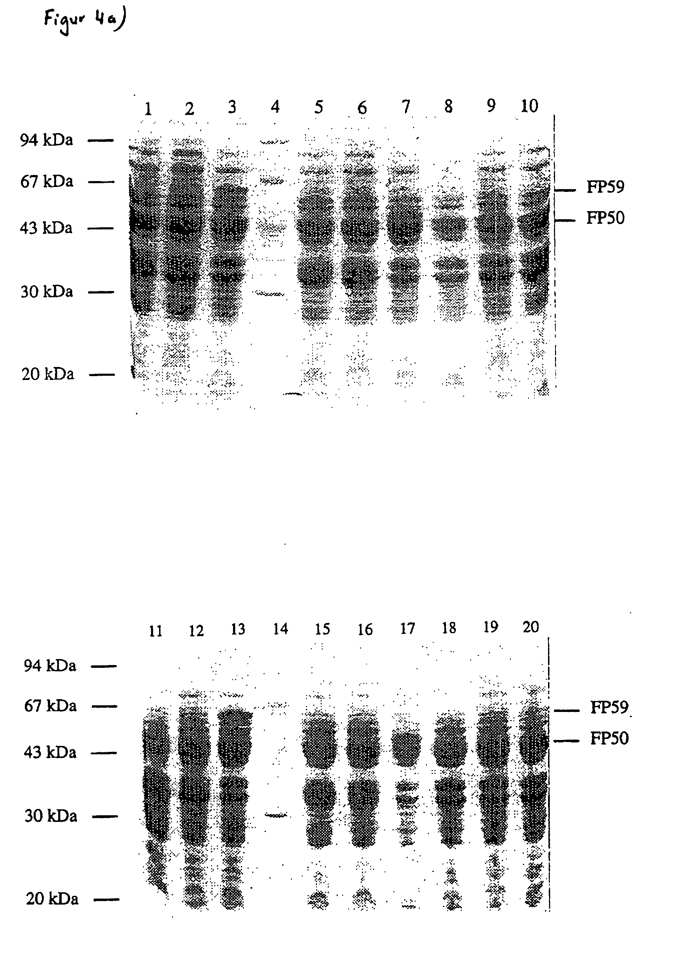

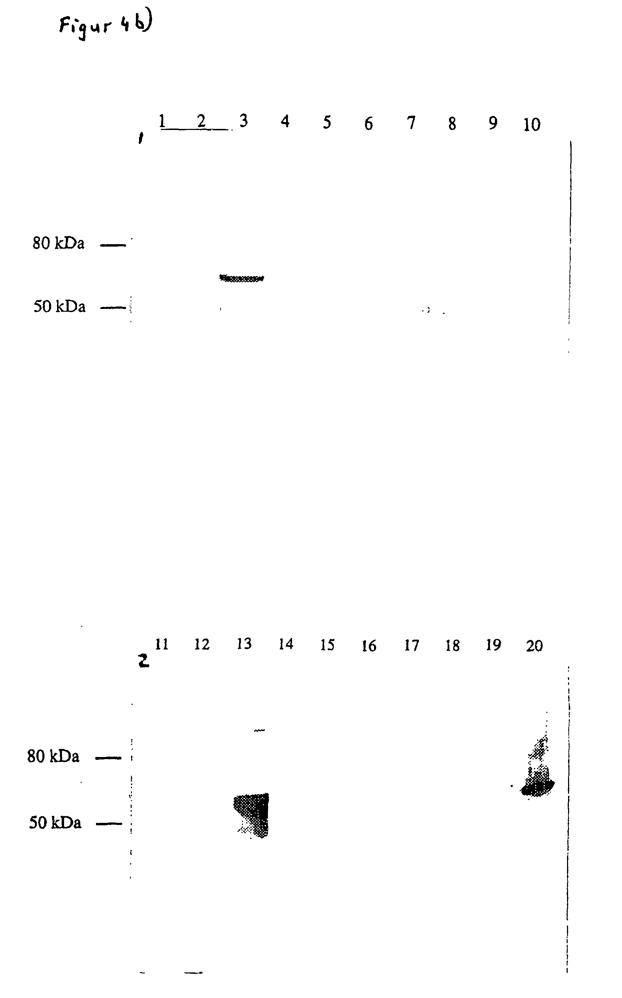

Die verglichen mit dem bisher existierenden System Iga-β substantiell stärkere Expression, die ohne die bei Iga-β zu beobachtende Neigung zur Lyse erzielt wurde, konnte eindeutig durch vergleichende Elektrophorese von Ganzzell-Lysaten (Figur 4a) demonstriert werden.

Der Nachweis der Oberflächenexposition von FP59 wurde durch verschiedene Methoden geführt. Die Proteasesensitivität von FP59 zeigte sich im Protein-Gel durch eine Verringerung des Molekulargewichts im Anschluß an einen Zusatz von Trypsin oder Chymotrypsin (Figur 3a). Es entstanden Protease-resistente Fragmente mit jeweils circa 33-35 kDa Masse (Figur 3a). Diese Protease-resistenten Fragmente enthalten keine immunogenen Anteile des Passagierproteins. Dies konnte durch Western-Blot-Analyse von Ganzzell-Lysaten unter Verwendung eines Anti-Choleratoxin B Serums im Vergleich von Protease-verdauten und unverdauten FP59-exprimierenden E.coli gezeigt werden (Figur 4b und Vergleich von 4a und 4b).

Durch N-terminale Ansequenzierung der membrangeschützten Trypsinverdauungsprodukte wurde gefunden, daß die Membranlinkerregion beim AIDA-Autotransporter eine Länge von 55 Aminosäuren besitzt.

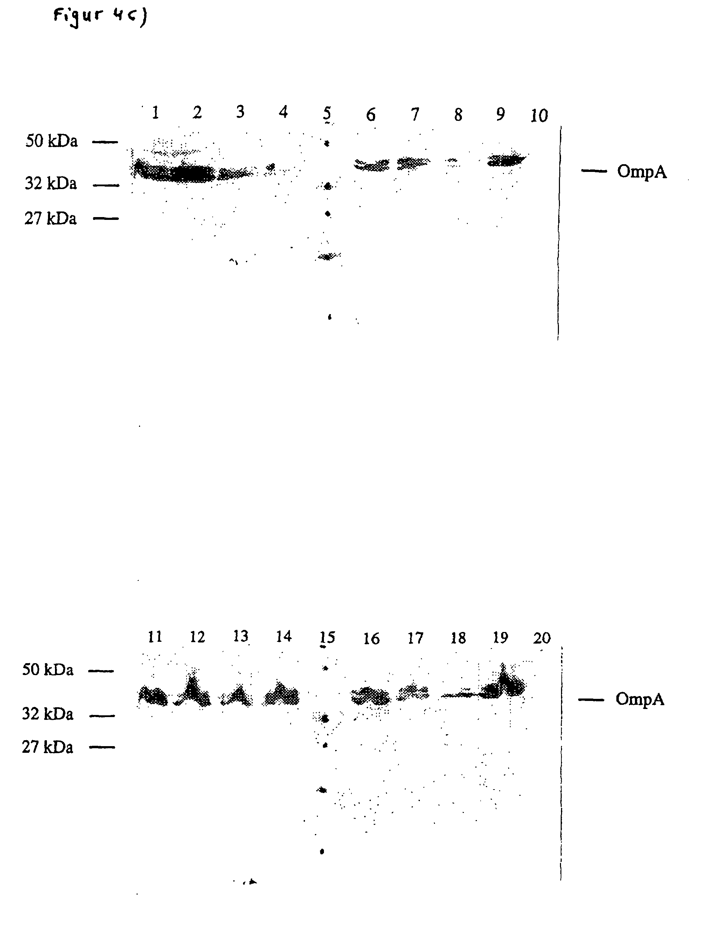

Mit den Proteaseverdauungen konnte auch die Integrität der Außenmembran von FP59-exprimierenden E.coli gezeigt werden (Abb. 4c). Dazu wurden Ganzzell-Lysate im Anschluß an den Verdau mit Trypsin durch Immunoblot mit einem Anti-OmpA-Serum entwickelt. Sowohl unverdaute Zellen als auch trypsinverdaute Zellen zeigten ein intaktes OmpA, wie es für Zellen mit einer intakten Außenmembran zu erwarten war.

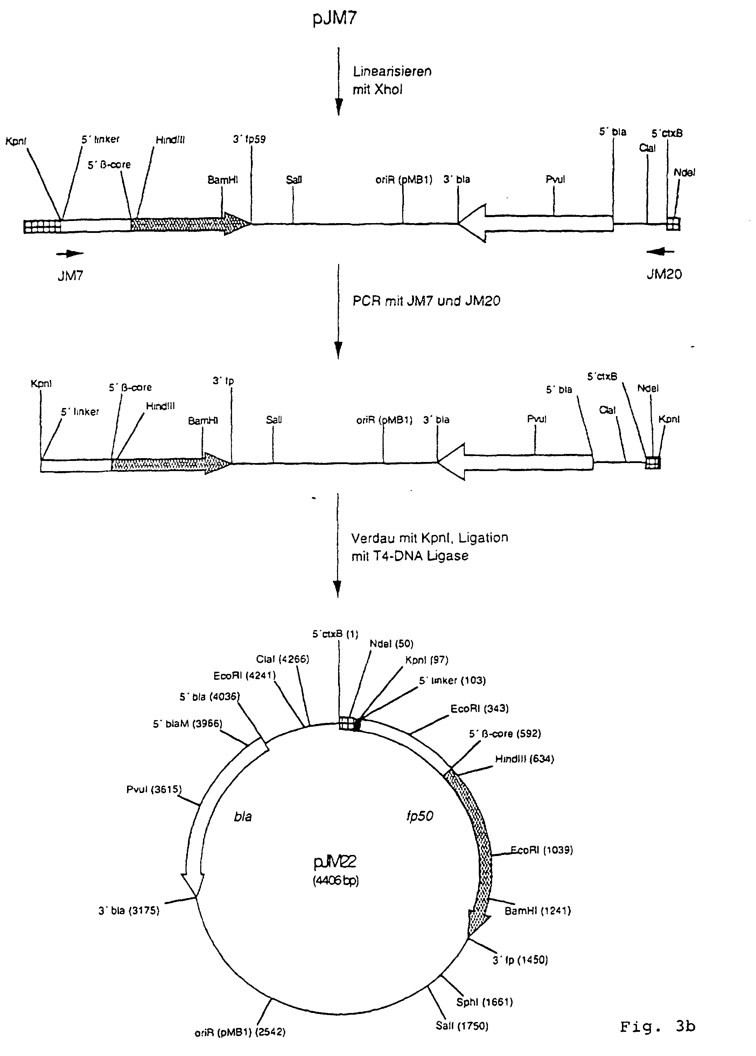

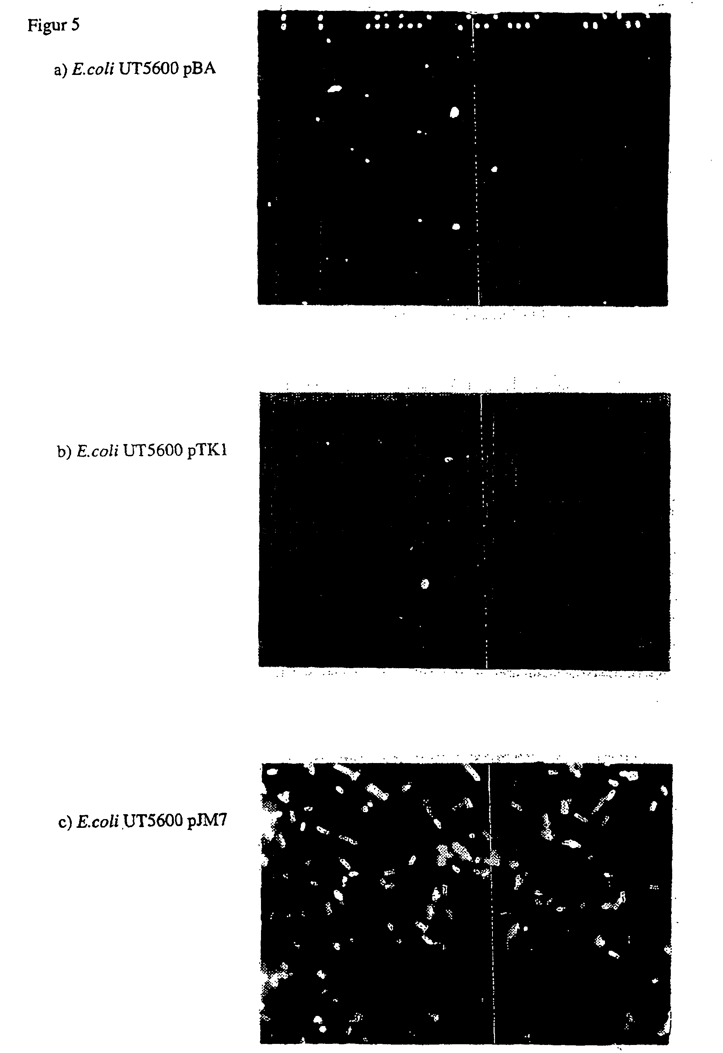

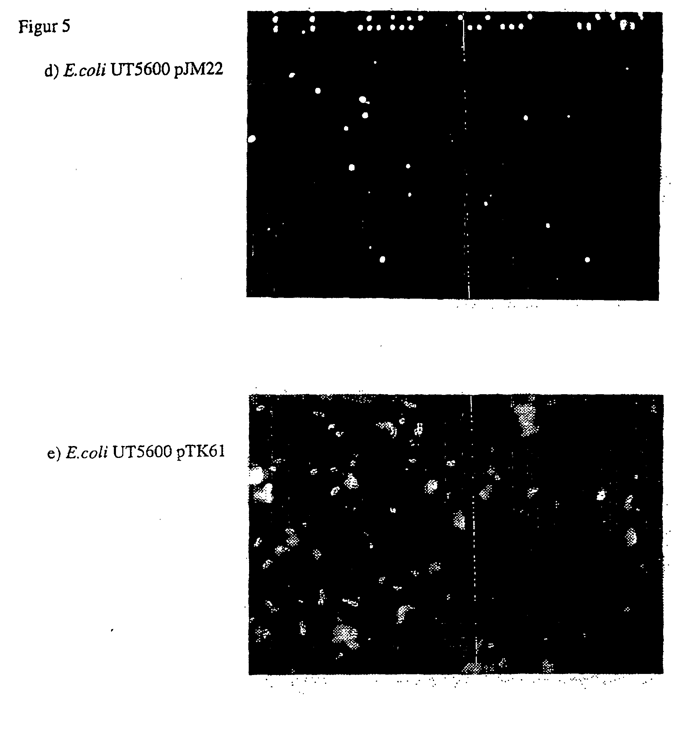

Auch mit Immunfluoreszenzstudien ließ sich die Oberflächenexposition und starke Expression von FP59 zeigen (Figur 5). Durch Bindung von fluoreszenzmarkierten Antikörpern läßt sich die Oberflächenexposition eines Antigens auf einer Bakteriezelle bei intakter Außenmembran nachweisen. Dies zeigten FP59 exprimierende E.coli-Zellen durch starke Fluoreszenz an. Die als Negativkontrollen verwendeten E.coli Zellen mit periplasmatisch exprimiertem Choleratoxin B, mit oberflächenexponiertem FP50 (Figur 3b) und mit nichtrekombinantem Klonierungsvektor waren eindeutig negativ. Das periplasmatische Choleratoxin B demonstrierte die Unzugänglichkeit des Periplasmas für Antikörper (Figur 5b), durch das negative Resultat der Immunfluoreszenz mit FP50 konnte eine Kreuzreaktivität des verwendeten Antiserums (gegen das Passagierprotein) mit den AIDA-Anteilen von FP59 ausgeschlossen werden (Figur 5d). Die Immunfluoreszenz mit dem nichtrekombinanten Klonierungsvektor war ein Maß für die der Meßmethode innewohnende Hintergrundfärbung (Figur 5a). Außerdem war somit ein Vergleich der Expression von FP59 mit B61, dem von pTK61 produzierten, oberflächenpräsentierten Choleratoxin B-Iga-β-Fusionsprotein möglich (Figuren 5c und 5e), wobei ebenfalls ein eindeutiger Vorteil des neuen erfindungsgemäßen Systems nachgewiesen werden konnte.

### Beispiel 3:

### Konstruktion einer oberflächenpräsentierten Peptidfusion

Ein Peptid, das als lineares Epitop für einen monoklonalen Antikörper (Dü142) fungiert, wurde auf der Oberfläche präsentiert und nachgewiesen. Zur Klonierung des Peptids wurde eine PCR-abhängige Strategie benutzt, die für die Generierung und Oberflächenexposition von Peptidbibliotheken äußerst geeignet ist. Dabei wird eine dreifache Genfusion aus dem Exportsignal von ctxB (Basen 1- 81), einer für ein Peptid kodierenden kurzen Sequenz (Basen 82-96) und der aida-linker/aida-β-Region (Basen 103-1450) gebildet.

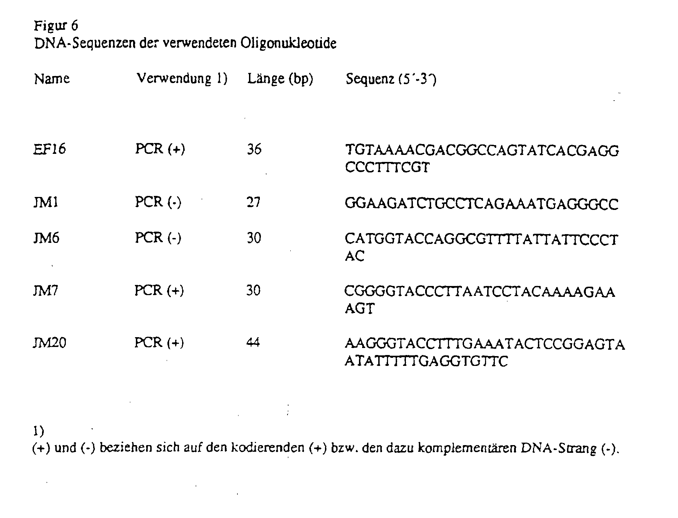

pJM7 (Figur 3a) wurde mit XhoI linearisiert und als Matrize für eine PCR mit den Oligonukleotiden JM7 und JM20 (Figur 6) benutzt (Figur 3b). Beide Oligonukleotide wiesen an ihren 5'-Enden eine KpnI-Erkennungssequenz auf. JM7 wurde so gewählt, daß bei seiner Verwendung in einer PCR die aida-linker/aida-β-Domänen amplifiziert wurden. JM20 wurde so gewählt, daß im PCR-Produkt die in ctxB enthaltene Signalsequenz für den Sec-abhängigen Membrantransport über die Cytoplasmamembran und die sechs daran anschließenden Codons mit enthalten waren. Außerdem enthielt JM20 in seiner 5'-ständigen, nicht zur Matrize komplementären Verlängerung, fünf Codons, die für das lineare Epitop des Antikörpers Dü142 kodierten. Stromaufwärts dieser Codons lag die KpnI-Erkennungssequenz. Nach der PCR wurde das resultierende Produkt mit KpnI hydrolysiert, mit sich selbst ligiert und anschließend in E.coli transformiert. Die Identifizierung korrekter Genfusionen wurde mittels Kolonie-Immunoblot vorgenommen (ohne Figur). Der Nachweis der Expression und der Oberflächenexposition wurde in Analogie zu den in Beispiel 2 beschriebenen Methoden durch Western-BIot-Analyse von Proteaseverdauungen und Analyse von Proteinfärbungen im Gel (Figuren 4a,b,c) geführt.

Die Generierung umfassender Peptidbibliotheken ist durch eine geringfügige Änderung der hier beschriebenen Klonierungsstrategie machbar. Die für JM20 beschriebene Aufteilung der verschiedenen funktionellen Bereiche dieses Oligonukleotids muß dazu so geändert werden, daß der für das lineare Epitop kodierende Bereich durch einen Bereich ersetzt wird, der bei der Oligonukleotidsynthese gewollt der Degeneration unterworfen wird. Degeneration heißt, daß anstatt definierter Basen an allen Positionen dieses funktionellen Bereichs einzelne, mehrere, oder alle Basen durch ein Basengemisch aus bis zu vier verschiedenen Basen ersetzt werden. Dadurch kann jedes Codon anstatt für eine Aminosäure für bis zu 20 verschiedene Aminosäuren kodieren, wodurch ein Pool von kodierenden Sequenzen ensteht, die für alle denkbaren Kombinationen von Aminsosäuren in einem Peptid der vorgegebenen Länge theoretisch möglich sind. Die Zelle, die das Peptid mit der gewünschten Eigenschaft trägt kann nun vermittelt durch Bindung des oberflächenexponierten Peptids an einen Bindungspartner, der beispielsweise an eine Matrix immobilisiert vorliegt, Fluoreszenz-markiert ist oder an Magnetkugeln gekoppelt ist, isoliert werden und zur beständigen Produktion und Charakterisierung verwendet werden.

## Patentansprüche

1. Verfahren zur Präsentation von Peptiden oder/und Polypeptiden auf der Oberfläche von Gram-negativen Wirtsbakterien,
wobei man,
(a) ein Wirtsbakterium bereitstellt, das transformiert mit einem Vektor, auf dem in operativer Verknüpfung mit einem Promotor eine fusionierte Nukleinsäuresequenz lokalisiert ist, umfassend:
(i) einen Signalpeptid-kodierenden Nukleinsäureabschnitt,
(ii) einen für das zu präsentierende Passagierpeptid oder/und Passagierpolypeptid kodierenden Nukleinsäureabschnitt,
(iii) gegebenenfalls einen für eine Proteaseerkennungsstelle kodierenden Nukleinsäureabschnitt,
(iv) einen für einen Transmembranlinker kodierenden Nukleinsäureabschnitt und
(v) einen für eine Transporterdomäne eines Autotransporters kodierenden Nukleinsäureabschnitt; und
(b) das Wirtsbakterium unter Bedingungen kultiviert, bei denen eine Expression der fusionierten Nukleinsäuresequenz und eine Präsentation des von dem Nukleinsäureabschnitt (ii) kodierten Peptids oder Polypeptids an der Oberfläche des Wirtsbakteriums erfolgt,
**dadurch gekennzeichnet,**
**daß** der Nukleinsäureabschnitt (ii) gegenüber dem für die Transporterdomäne (v) kodierenden Nukleinsäureabschnitt heterolog und das Wirtsbakterium gegenüber dem für die Transporterdomäne (v) kodierenden Nukleinsäureabschnitt homolog ist.

2. Verfahren nach Anspruch 1,
**dadurch gekennzeichnet,**
**daß** der verwendete Autotransporter aus einer Gattung der Enterobacterioceae abgeleitet wurde und in einem Wirtsbakterium einer Gattung der Enterobacterioceae verwendet wird.

3. Verfahren nach Anspruch 1 oder 2,
**dadurch gekennzeichnet,**
**daß** man die Transporterdomäne des Aida-Proteins aus E.coli oder eine Variante davon verwendet, wobei die Variante eine Homologie von mindestens 80 % zu der Transporterdomäne zumindest im Bereich der β-Faltblattstrukturen hat.

4. Verfahren nach Anspruch 1 oder 2,
**dadurch gekennzeichnet,**
**daß** man die Transporterdomäne des SepA-Proteins aus Shigella flexneri oder eine Variante davon verwendet, wobei die Variante eine Homologie von mindestens 80 % zu der Transporterdomäne zumindest im Bereich der β-Faltblattstrukturen hat.

5. Verfahren nach Anspruch 1 oder 2,
**dadurch gekennzeichnet,**
**daß** man die Transporterdomäne des IcsA-Proteins aus Shigella flexneri oder eine Variante davon verwendet, wobei die Variante eine Homologie von mindestens 80 % zu der Transporterdomäne zumindest im Bereich der β-Faltblattstrukturen hat.

6. Verfahren nach Anspruch 2,
**dadurch gekennzeichnet,**
**daß** man die Transporterdomäne des Tsh-Proteins aus E.coli oder eine Variante davon verwendet, wobei die Variante eine Homologie von mindestens 80 % zu der Transporterdomäne zumindest im Bereich der β-Faltblattstrukturen hat.

7. Verfahren nach Anspruch 2,
**dadurch gekennzeichnet,**
**daß** man die Transporterdomäne des Ssp-Proteins aus Serratin marcescens oder eine Variante davon verwendet, wobei die Variante eine Homologie von mindestens 80 % zu der Transporterdomäne zumindest im Bereich der β-Faltblattstrukturen hat.

8. Verfahren nach Anspruch 1,
**dadurch gekennzeichnet,**
**daß** man die Transporterdomäne des Hsr-Proteins aus Helicobacter mustelae, des Prn-Proteins aus Bordetella ssp., des Hap-Proteins aus Haemophilus influenzae, des BrkA-Proteins aus Bordetella pertussis, des VacA-Proteins aus Helicobacter pylori oder eines der Rickettsienproteine 190kDa Zelloberflächenprotein, SpaP, rOmpB oder SlpT, verwendet.

9. Verfahren nach einem der Ansprüche 1 - 8,
**dadurch gekennzeichnet,**
**daß** ein oder mehrere Peptide, insbesondere Peptide mit einer Länge von 4-50 Aminosäuren präsentiert werden.

10. Verfahren nach einem der Ansprüche 1 - 8,
**dadurch gekennzeichnet,**
**daß** ein oder mehrere eukaryontische Polypeptide präsentiert werden.

11. Verfahren nach Anspruch 10,
**dadurch gekennzeichnet,**
**daß** es sich bei dem Passagierpolypeptid um einen Antikörper oder eine Antigen bindende Domäne eines Antikörpers handelt, wobei Antigen bindende Domäne mindestens den Bereich eines Antikörpermoleküls bezeichnet, der hinreichend ist für die spezifische Bindung eines Antigens.

12. Verfahren nach Anspruch 10,
**dadurch gekennzeichnet,**
**daß** es sich bei dem Passagierpolypeptid um die α-Kette eines MHC Klasse II Moleküls handelt.

13. Verfahren nach Anspruch 10,
**dadurch gekennzeichnet,**
**daß** es sich bei dem Passagierpolypeptid um die β-Kette eines MHC-Klasse II Moleküls handelt.

14. Verfahren nach Anspruch 13,
**dadurch gekennzeichnet,**
**daß** es sich bei dem Passagierpolypeptid um die β-Kette eines MHC Klasse II Moleküls handelt, an dem N-terminal Aminosäuren angehängt vorliegen, die sich als Peptid in die Bindungsgrube des funktionellen MHC Moleküls einlagern können.

15. Verfahren nach einem der Ansprüche 1 -14,
**dadurch gekennzeichnet,**
**daß** Bibliotheken von varianten Passagierpeptiden oder -polypeptiden erzeugt, in Wirtszellen exprimiert und an der Oberfläche präsentiert werden.

16. Verfahren nach einem der Ansprüche 1 - 15,
**dadurch gekennzeichnet,**
**daß** eine Wirtsbakterienzelle unterschiedliche Passagierpeptide oder -polypeptide jeweils in Verbindung mit einer Transporterdomäne präsentiert.

17. Verfahren nach Anspruch 16,
**dadurch gekennzeichnet,**
**daß** unterschiedliche Transporterdomänen in Verbindung mit unterschiedlichen Passagierpeptiden oder -polypeptiden verwendet werden.

18. Verfahren nach einem der Ansprüche 15 - 17 , weiterhin umfassend den Schritt des Selektionierens einzelner Passagierpeptide oder -polypeptide aus einer Bibliothek von varianten Peptiden oder Polypeptiden.

19. Verfahren zur Herstellung einer gegebenenfalls Varianten Population von oberflächenexponierten Peptiden oder Polypeptiden und zur Identifizierung der Bakterien, die Peptide bzw. Polypeptide mit einer jeweils gewünschten Eigenschaft tragen, wobei das Verfahren folgende Schritte umfaßt:
(1) Herstellen eines oder mehrerer Fusionsgene durch Klonierung der kodierenden Sequenz eines gewünschten Passagiers in frame mit der kodierenden Sequenz der Transporterdomäne des ATDA-Proteins aus E.coli oder einer Variante davon, wobei die Variante eine Homologie von mindestens 80 % zu der Transporterdomäne zumindest im Bereich der β-Faltblattstrukturen hat, und eines Signalpeptides in mindestens einen Vektor;
(2) gegebenenfalls Variieren des Passagierpeptids bzw. -polypeptids durch Mutagenese;
(3) Einbringen des Vektors oder der Vektoren in Wirtsbakterien, die den Passagier oder die Passagiere stabil an der Oberfläche präsentieren können,
(4) Exprimieren des Fusionsgens bzw. der Fusionsgene in den Wirtsbakterien;
(5) Kultivieren der Bakterien zur Produktion des stabil oberflächenexponiert präsentierten Passagiers oder der stabil oberflächenexponiert präsentierten Passagiere;
(6) gegebenenfalls Selektionieren der Bakterien, die den Passagier oder die Passagiere mit den gewünschten Eigenschaften auf der Oberfläche tragen, und
(7) Gegebenenfalls Charakterisieren eines Bindungspartners für den Passagier mit den optimalen Eigenschaften.

20. Verfahren nach Anspruch 19, wobei das Verfahren mehrmals durchlaufen wird.

21. Verfahren nach einem der Ansprüche 19 - 20, wobei das im Fusionsprotein enthaltene Passagierprotein ein Peptid oder Polypeptid mit einer Affinität zu einem Bindungspartner, ein Ligand, ein Rezeptor, ein Antigen, ein Toxin-bindendes Protein, ein Protein mit enzymatischer Aktivität, ein Nukleinsaure-bindendes Protein, ein Inhibitor, ein Chelator-Eigenschaften habendes Protein, ein Antikörper oder eine Antigen-bindende Domäne eines Antikörpers ist.

22. Verfahren nach einem der Ansprüche 19 - 21, wobei die Identifizierung des Bakteriums, das einen Passagier mit einer gewünschten Bindungsaffinität oberflächenexponiert präsentiert, durch Bindung an einen immobilisierten oder/und markierten Bindungspartner erfolgt.

23. Verfahren nach Anspruch 19, wobei der Bindungspartner so modifiziert ist, daß er in einem zweiten Schritt durch einen für die Modifikation spezifischen Bindungspartner detektiert werden kann.

24. Verfahren nach einem der Ansprüche 1 - 23,
**dadurch gekennzeichnet,**
**daß** Passagierproteine oder Teile davon auf der Bakterienoberfläche chemisch oder enzymatisch modifiziert werden.

25. Verfahren nach Anspruch 24,
**dadurch gekennzeichnet,**
**daß** die Modifikation eine nicht-kovalente Modifikation ist.

26. Verfahren nach Anspruch 24,
**dadurch gekennzeichnet,**
**daß** die Modifikation eine kovalente Modifikation ist.

27. Verfahren nach Anspruch 26,
**dadurch gekennzeichnet,**
**daß** die Modifikation eine Glykosylierung ist.

28. Verfahren nach Anspruch 26,
**dadurch gekennzeichnet,**
**daß** die Modifikation eine Phosphorylierung ist.

29. Verfahren nach Anspruch 24,
**dadurch gekennzeichnet,**
**daß** die Modifikation eine Proteolyse ist.

30. Verfahren nach Anspruch 29,
**dadurch gekennzeichnet,**
**daß** Passagierproteine oder Teile davon durch intrinsische oder extern zugeführte Proteasen selektiv von der Bakterienoberfläche freigesetzt werden.

31. Verfahren nach Anspruch 30,
**dadurch gekennzeichnet,**
**daß** Passagierproteine oder Teile davon durch eine intrinsische Protease der Wirtszelle, insbesondere die OmpT-Protease, die OmpK-Protease oder die Protease X, freigesetzt werden.

32. Verfahren nach Anspruch 31,
**dadurch gekennzeichnet,**
**daß** Passagierproteine oder Teile davon durch eine extern zugeführte Protease, insbesondere die IgA-Protease, Thrombin oder Faktor X, freigesetzt werden.

33. Rekombinanter Vektor auf dem in operativer Verknüpfung mit einem Promotor eine fusionierte Nukleinsäuresequenz lokalisiert ist, umfassend:
(i) einen Signalpeptid-kodierenden Nukleinsäureabschnitt,
(ii) einen für das zu präsentierende Passagierpeptid oder/und Passagierpolypeptid kodierenden Nukleinsäureabschnitt,
(iii) gegebenenfalls einen für eine Proteaseerkennungsstelle kodierenden Nukleinsäureabschnitt,
(iv) einen für einen Transmembranlinker kodierenden Nukleinsäureabschnitt und
(v) einen für die Transporterdomäne des AIDA-Proteins aus E.coli oder für eine Variante davon kodierenden Nukleinsäureabschnitt, wobei die Variante eine Homologie von mindestens 80 % zu der Transporterdomäne zumindest im Bereich der β-Faltblattstrukturen hat
wobei der Nukleinsäureabschnitt (ii) gegenüber dem für die Transporterdomäne (v) kodierenden Nukleinsäureabschnitt heterolog ist.

34. Rekombinantes Gram-negatives Wirtsbakterium,
**dadurch gekennzeichnet,**
**daß** es mit einem Vektor nach Anspruch 33 transformiert ist.

35. Rekombinantes Gram-negatives Wirtsbakterium, das transformiert ist mit einem rekombinanten Vektor auf dem in operativer Verknüpfung mit einem Promotor eine fusionierte Nukleinsäuresequenz lokalisiert ist, umfassend:
(i) einen Signalpeptid-kodierenden Nukleinsäureabschnitt,
(ii) einen für das zu präsentierende Passagierpeptid oder/und Passagierpolypeptid kodierenden Nukleinsäureabschnitt,
(iii) gegebenenfalls einen für eine Proteaseerkennung s-stelle kodierenden Nukleinsäureabschnitt,
(iv) einen für einen Transmembranlinker kodierenden Nukleinsäureabschnitt und
(v) einen für eine Transporterdomäne eines Autotransporters kodierenden Nukleinsäureabschnitt;
wobei der Nukleinsäureabschnitt (ii) gegenüber dem für die Transporterdomäne (v) kodierenden Nukleinsäureabschnitt heterolog ist und wobei das Wirtsbakterium gegenüber dem für die Transporterdomäne (v) kodierenden Nukleinsäureabschnitt homolog ist.

36. Wirtsbakterium nach Anspruch 35,
**dadurch gekennzeichnet,**
**daß** es eine E.coli Zelle ist.

37. Wirtsbakterium nach einem der Ansprüche 35 - 36,
**dadurch gekennzeichnet,**
**daß** der Nukleinsäureabschnitt (v) für die Transporterdomäne des AIDA-Proteins oder eine Variante davon kodiert, wobei die Variante eine Homologie von mindestens 80 % zu der Transporterdomäne zumindest im Bereich der β-Faltblattstrukturen hat.

## Claims

1. Process for presenting peptides or/and polypeptides on the surface of gram-negative host bacteria, in which
a) a host bacterium is provided which is transformed with a vector on which a fused nucleic acid sequence is located in operative linkage with a promoter, said sequence comprising
(i) a nucleic acid section coding for a signal peptide
(ii) a nucleic acid section coding for the passenger peptide or/and passenger polypeptide to be presented,
(iii) optionally a nucleic acid section coding for a protease recognition site,
(iv) a nucleic acid section coding for a transmembrane linker and
(v) a nucleic acid section coding for a transporter domain of an autotransporter; and
b) the host bacterium is cultured under conditions which allow expression of the fused nucleic acid sequence and presentation of the peptide or polypeptide encoded by the nucleic acid section (ii) on the surface of the host bacterium,
**characterized in that** the nucleic acid section (ii) is heterologous with respect to the nucleic acid section coding for the transporter domain (v) and the host bacterium is homologous with respect to the nucleic acid section coding for the transporter domain (v).

2. Process as claimed in claim 1, **characterized in that** the autotransporter used has been derived from a genus of enterobacterioceae and is used in a host bacterium of a genus of enterobacterioceae.

3. Process as claimed in claim 1 or 2, **characterized in that** the transporter domain of the AIDA protein from E. coli or a variant thereof is used, the variant having a homology of at least 80 % to the transporter domain at least in the region of the β-pleated sheet structures.

4. Process as claimed in claim 1 or 2, **characterized in that** the transporter domain of the SepA protein from Shigella flexneri or a variant thereof is used, the variant having a homology of at least 80 % to the transporter domain at least in the region of the β-pleated sheet structures.

5. Process as claimed in claim 1 or 2, **characterized in that** the transporter domain of the IcsA protein from Shigella flexneri or a variant thereof is used, the variant having a homology of at least 80 % to the transporter domain at least in the region of the β-pleated sheet structures.

6. Process as claimed in claim 2, **characterized in that** the transporter domain of the Tsh protein from E. coli or a variant thereof is used, the variant having a homology of at least 80 % to the transporter domain at least in the region of the β-pleated sheet structures.

7. Process as claimed in claim 2, **characterized in that** the transporter domain of the Ssp protein from Serratin marcescens or a variant thereof is used, the variant having a homology of at least 80 % to the transporter domain at least in the region of the β-pleated sheet structures.

8. Process as claimed in claim 1, **characterized in that** the transporter domain of the Hsr protein from helicobacter mustelae, of the Pm protein from Bordetella ssp., of the Hap protein from Haemophilus influenzae, of the BrkA protein from Bordetella pertussis, of the VacA protein from Helicobacter pylori or of one of the rickettsial proteins, 190 kDa cell surface protein, SpaP, rOmpB or SlpT is used.

9. Process as claimed in one of the claims 1-8, **characterized in that** one or more peptides, in particular peptides having a length of 4-50 amino acids are presented.

10. Process as claimed in one of the claims 1-8, **characterized in that** one or more eukaryotic polypeptides are presented.

11. Process as claimed in claim 10, **characterized in that** the passenger polypeptide is an antibody or an antigen-binding domain of an antibody, where antigen-binding domain refers to at least the region of an antibody molecule which is sufficient for specific binding of an antigen.

12. Process as claimed in claim 10, **characterized in that** the passenger polypeptide is the α chain of an MHC class II molecule.

13. Process as claimed in claim 10, **characterized in that** the passenger polypeptide is the β chain of an MHC class II molecule.

14. Process as claimed in claim 13, **characterized in that** the passenger polypeptide is the β chain of an MHC class II molecule to the N-terminus of which amino acids are attached which as a peptide can insert themselves into the binding cavity of the functional MHC molecule.

15. Process as claimed in one of the claims 1-14, **characterized in that** libraries of variant passenger peptides or polypeptides are produced, expressed in host cells and presented on the surface.

16. Process as claimed in one of the claims 1-15, **characterized in that** a host bacterial cell presents different passenger peptides or polypeptides in each case connected to a transporter domain.

17. Process as claimed in claim 16, **characterized in that** different transporter domains are used connected to different passenger peptides or polypeptides.

18. Process as claimed in one of the claims 15-17 further comprising the step of selecting single passenger peptides or polypeptides from a library of variant peptides or polypeptides.

19. Process for producing an optionally variant population of surface-exposed peptides or polypeptides and for identifying bacteria which carry the peptides or polypeptides each having a desired property, said process comprising the following steps:
(1) producing one or more fusion genes by cloning the coding sequence of a desired passenger in frame with the coding sequence of the transporter domain of the AIDA protein from E. coli or of a variant thereof the variant having a homology of at least 80 % to the transporter domain at least in the regions of the β-pleated sheet structures, and of a signal peptide into at least one vector;
(2) optionally varying the passenger peptide or polypeptide by mutagenesis;
(3) introducing the vector or vectors into host bacteria that can stably present the passenger or passengers on the surface;
(4) expressing the fusion gene or fusion genes in the host bacteria;
(5) culturing the bacteria to produce the passenger presented stably exposed on the surface or the passengers presented stably exposed on the surface;
(6) optionally selecting the bacteria which carry the passenger or passengers having the desired properties on the surface and
(7) optionally characterizing a binding partner for the passenger having the optimal properties.

20. Process as claimed in claim 19, in which the process is performed several times.

21. Process as claimed in one of the claims 19-20, in which the passenger protein contained in the fusion protein is a peptide or polypeptide having an affinity for a binding partner, a ligand, a receptor, an antigen, a toxin-binding protein, a protein having enzymatic activity, a nucleic acid-binding protein, an inhibitor, a protein having chelator properties, an antibody or an antigen-binding domain of an antibody.

22. Process as claimed in claims 20-24, in which the bacterium which presents a surface-exposed passenger having a desired binding affinity is identified by binding to an immobilized or/and labelled binding partner.

23. Process as claimed in claim 20, in which the binding partner is modified in such a manner that it can be detected in a second step by a binding partner specific for the modification.

24. Process as claimed in one of the claims 1-23, **characterized in that** passenger proteins or parts thereof are chemically or enzymatically modified on the bacterial surface.

25. Process as claimed in claim 24, **characterized in that** the modification is a non-covalent modification.

26. Process as claimed in claim 24, **characterized in that** the modification is a covalent modification.

27. Process as claimed in claim 26, **characterized in that** the modification is a glycosylation.

28. Process as claimed in claim 26, **characterized in that** the modification is a phosphorylation.

29. Process as claimed in claim 24, **characterized in that** the modification is a proteolysis.

30. Process as claimed in claim 29, **characterized in that** passenger proteins or parts thereof are selectively released from the bacterial surface by intrinsic or externally added proteases.

31. Process as claimed in claim 30, **characterized in that** passenger proteins or parts thereof are released by an intrinsic protease of the host cell, in particular OmpT protease, OmpK protease or protease X.

32. Process as claimed in claim 31, **characterized in that** passenger proteins or parts thereof are released by an externally added protease, in particular IgA protease, thrombin or factor X.

33. Recombinant vector on which a fused nucleic acid sequence operatively linked to a promoter is located, comprising:
(i) a signal peptide-encoding nucleic acid section,
(ii) a nucleic acid section coding for the passenger peptide or/and passenger polypeptide to be presented
(iii) optionally a nucleic acid section coding for a protease recognition site,
(iv) a nucleic acid section coding for a transmembrane linker and
(v) a nucleic acid section coding for the transporter domain of the AIDA protein from E. coli or for a variant thereof, the variant having a homology of at least 80 % to the transporter domain at least in the region of the β-pleated sheet structures,
wherein the nucleic acid section (ii) is heterologous with respect to the nucleic acid section coding for the transporter domain (v).

34. Recombinant gram-negative host bacterium, **characterized in that** it is transformed with a vector as claimed in claim 33.

35. Recombinant gram-negative host bacterium which is transformed with a recombinant vector on which a fused nucleic acid sequence is located in operative linkage with a promoter, said sequence comprising:
(i) a signal peptide-encoding nucleic acid section,
(ii) a nucleic acid section coding for the passenger peptide or/and passenger polypeptide to be presented
(iii) optionally a nucleic acid section coding for a protease recognition site,
(iv) a nucleic acid section coding for a transmembrane linker and
(v) a nucleic acid section coding for a transporter domain of an autotransporter,
wherein the nucleic acid section (ii) is heterologous with respect to the nucleic acid section (v) coding for the transporter domain and wherein the host bacterium is homologous with respect to the nucleic acid section (v) coding for the transporter domain.

36. Host bacterium as claimed in claim 35, **characterized in that** it is an E. coli cell.

37. Host bacterium as claimed in one of the claims 35-36, **characterized in that** the nucleic acid section (v) codes for the transporter domain of the AIDA protein or a variant thereof, the variant having a homology of at least 80 % to the transporter domain at least in the region of the β-pleated sheet structures.

## Revendications

1. Procédé de présentation de peptides ou/et de polypeptides à la surface de bactéries-hôtes gram-négatives, tel que l'on :
a) prépare une bactérie-hôte par transformation avec un vecteur sur lequel une séquence d'acide nucléique de fusion est localisée en liaison opératoire avec un promoteur, comprenant :
(i) un fragment d'acide nucléique codant pour un peptide signal,
(ii) un fragment d'acide nucléique codant pour le peptide passager et/ou le polypeptide passager à présenter,
(iii) le cas échéant un fragment d'acide nucléique codant pour un site de reconnaissance d'une protéase,
(iv) un fragment d'acide nucléique codant pour un lieur transmembranaire et
(v) un fragment d'acide nucléique codant pour un domaine transporteur d'un autotransporteur ; et
b) cultive la bactérie-hôte dans des conditions dans lesquelles a lieu une expression de la séquence d'acide nucléique de fusion et une présentation en surface de la bactérie-hôte du peptide ou polypeptide codé par le fragment (ii) d'acide nucléique,
**caractérisé en ce que** :
le fragment d'acide nucléique (ii) est hétérologue par rapport au fragment d'acide nucléique codant pour le domaine transporteur (v) et la bactérie-hôte est homologue par rapport au fragment d'acide nucléique codant le domaine transporteur (v).

2. Procédé selon la revendication 1, **caractérisé en ce que** l'autotransporteur utilisé provient d'une espèce d'Enterobacterioceae et est utilisé dans une bactérie-hôte d'une espèce d'Enterobacterioceae.

3. Procédé selon la revendication 1 ou 2, **caractérisé en ce qu'**on utilise le domaine transporteur de la protéine AIDA d'*E.coli* ou une variante de celle-ci ; ladite variante ayant une homologie d'au moins 80% avec le domaine transporteur au moins dans la région des structures feuillets plissés bêta.

4. Procédé selon la revendication 1 ou 2, **caractérisé en ce qu'**on utilise le domaine transporteur de la protéine SepA de *Shigella flexneri* ou une variante de celle-ci ; ladite variante ayant une homologie d'au moins 80% avec le domaine transporteur au moins dans la régions des structures feuillets plissés bêta.

5. Procédé selon la revendication 1 ou 2, **caractérisé en ce qu'**on utilise le domaine transporteur de la protéine IcsA de *Shigella flexneri* ou une variante de celle-ci, ladite variante ayant une homologie d'au moins 80% avec le domaine transporteur au moins dans la région des structures feuillets plissés bêta.

6. Procédé selon la revendication 2, **caractérisé en ce qu'**on utilise le domaine transporteur de la protéine Tsh d'*E.coli* ou une variante de celle-ci, ladite variante ayant une homologie d'au moins 80% avec le domaine transporteur au moins dans la région des structures feuillets plissés bêta.

7. Procédé selon la revendication 2, **caractérisé en ce qu'**on utilise le domaine transporteur de la protéine Ssp de *Serratin marcescens* ou une variante de celle-ci, ladite variante ayant une homologie d'au moins 80% avec le domaine transporteur au moins dans la région des structures feuillets plissés bêta.

8. Procédé selon la revendication 1, **caractérisé en ce qu'**on utilise le domaine transporteur de la protéine Hsr d'*Helicobacter mustelae*, de la protéine Prn de *Bordetella ssp*., de la protéine Hap d'*Haemophilus influenzae*, de la protéine BrkA de *Bordetella pertussis,* de la protéine VacA d'*Helicobacter pylori* ou d'une des protéines de Rickettsia 190 kDa, protéine de surface, SpaP, rOmpB ou SlpT.

9. Procédé selon l'une quelconque des revendications 1 à 8, **caractérisé en ce qu'**un ou plusieurs peptides, en particulier des peptides d'une longueur de 4 à 50 acides aminés, sont présentés.

10. Procédé selon l'une quelconque des revendications 1 à 8, **caractérisé en ce qu'**une ou plusieurs polypeptides eucaryotes sont présentés.

11. Procédé selon la revendication 10, **caractérisé en ce que** le polypeptide passager est un anticorps ou un domaine liant l'antigène d'un anticorps, le domaine liant l'antigène caractérisant au moins la portion d'une molécule d'anticorps qui est suffisante pour la liaison spécifique d'un antigène.

12. Procédé selon la revendication 10, **caractérisé en ce que** le polypeptide passager est la chaîne α d'une molécule MHC de classe II.

13. Procédé selon la revendication 10, **caractérisé en ce que** le polypeptide passager est la chaîne β d'une molécule MHC de classe II.

14. Procédé selon la revendication 13, **caractérisé en ce que** le polypeptide passager est la chaîne β d'une molécule MHC de classe II, sur la partie N-terminale de laquelle sont ancrés des acides aminés, et qui peuvent en tant que peptides se loger dans le site de liaison de la molécule MHC fonctionnelle.

15. Procédé selon l'une quelconque des revendications 1 à 14, **caractérisé en ce que** des banques de variantes de peptides passagers ou polypeptides passagers sont créées, exprimées dans des cellules-hôtes et présentées à la surface.

16. Procédé selon l'une quelconque des revendications 1 à 15, **caractérisé en ce qu'**une cellule de bactérie-hôte présente différents peptides ou polypeptides passagers respectivement en liaison avec un domaine transporteur.

17. Procédé selon la revendication 16, **caractérisé en ce que** différents domaines transporteurs sont utilisés en liaison avec différents peptides ou polypeptides passagers.

18. Procédé selon l'une quelconque des revendications 15 à 17, comprenant en outre l'étape de sélection de peptides ou polypeptides passagers particuliers à partir d'une banque de variants de peptides ou polypeptides.

19. Procédé de fabrication d'une population variante le cas échéant de peptides ou polypeptides exposés en surface et d'identification des bactéries portant des peptides ou des polypeptides avec respectivement la propriété souhaitée, ledit procédé comprenant les étapes suivantes :
(1) Fabrication d'un ou plusieurs gènes de fusion par clonage de la séquence codante d'un passager souhaité en phase avec la séquence codante du domaine transporteur de la protéine AIDA d'*E.coli* ou d'une variante de celle-ci, ladite variante ayant une homologie d'au moins 80% avec le domaine transporteur au moins dans la région des structures feuillets plissés bêta et d'un peptide signal dans au moins un vecteur ;
(2) Le cas échéant la variation du peptide passager ou du polypeptide passager par mutagenèse ;
(3) L'apport du vecteur ou des vecteurs dans des bactéries-hôtes qui peuvent présenter le passager ou les passagers de façon stable à la surface ;
(4) Expression du gène de fusion ou des gènes de fusion des bactéries-hôtes ;
(5) Culture des bactéries pour produire le passager présenté exposé à la surface de façon stable ou des passagers présentés exposés à la surface de façon stable ;
(6) Le cas échéant sélection des bactéries qui portent en surface le ou les passagers ayant les propriétés souhaitées, et
(7) Le cas échéant caractérisation d'un partenaire de liaison pour le passager avec les propriétés optimales.

20. Procédé selon la revendication 19 tel que le procédé est réalisé plusieurs fois.

21. Procédé selon l'une quelconque des revendications 19 à 20, tel que la protéine passagère contenue dans la protéine de fusion est un peptide ou polypeptide ayant une affinité avec un partenaire de liaison, un ligand, un récepteur, un antigène, une protéine se liant à une toxine, une protéine avec une activité enzymatique, une protéine se liant aux acides nucléiques, un inhibiteur, une protéine ayant des propriétés chélatrices, un anticorps ou un domaine d'un anticorps se liant à l'antigène.

22. Procédé selon l'une quelconque des revendications 19 à 21, tel que l'identification de la bactérie qui présente un passager ayant une affinité de liaison souhaitée exposé à la surface, est réalisée par liaison avec un partenaire de liaison immobilisé ou/et marqué.

23. Procédé selon la revendication 19, tel que le partenaire de liaison est modifié de sorte que dans une deuxième étape il peut être détecté par l'intermédiaire d'un partenaire de liaison spécifique à la modification.

24. Procédé selon l'une quelconque des revendications 1 à 23, **caractérisé en ce que** des protéines passagères ou des parties de celles-ci sont modifiées de façon chimique ou enzymatique sur la surface des bactéries.

25. Procédé selon la revendication 24 **caractérisé en ce que** la modification est une modification non covalente.

26. Procédé selon la revendication 24, **caractérisé en ce que** la modification est une modification covalente.

27. Procédé selon la revendication 26, **caractérisé en ce que** la modification est une glycosylation.

28. Procédé selon la revendication 26, **caractérisé en ce que** la modification est une phosphorylation.

29. Procédé selon la revendication 24, **caractérisé en ce que** la modification est une protéolyse.

30. Procédé selon la revendication 29 **caractérisé en ce que** des protéines passagères ou des parties de celle-ci sont libérées sélectivement de la surface des bactéries via des protéases intrinsèques ou amenées par voie externe.

31. Procédé selon la revendication 30 **caractérisé en ce que** des protéines passagères ou des parties de celles-ci sont libérées via une protéase intrinsèque de la cellule-hôte, en particulier la protéase OmpT, la protéase OmpK ou la protéase X.

32. Procédé selon la revendication 31 **caractérisé en ce que** des protéines passagères ou des parties de celles-ci sont libérées via une protéase amenée par voie externe, en particulier l'IgA protéase, la thrombine ou le facteur X.

33. Vecteur recombinant sur lequel est localisée une séquence d'acide nucléique de fusion en liaison opératoire avec un promoteur, comprenant :
(i) un fragment d'acide nucléique codant pour un peptide signal,
(ii) un fragment d'acide nucléique codant pour le peptide passager ou/et le polypeptide passager à présenter,
(iii) le cas échéant un fragment d'acide nucléique codant pour un site de reconnaissance d'une protéase,
(iv) un fragment d'acide nucléique codant pour un lieur transmembranaire et
(v) un fragment d'acide nucléique codant pour le domaine transporteur de la protéine AIDA d'*E.coli* ou d'une variante de celle-ci, ladite variante ayant une homologie d'au moins 80% avec le domaine transporteur au moins dans la région des structures feuillets plissés bêta, le fragment d'acide nucléique (ii) étant hétérologue par rapport au fragment d'acide nucléique codant pour le domaine transporteur (v).

34. Bactérie-hôte gram-négative recombinante, **caractérisée en ce qu'**elle est transformée avec un vecteur selon la revendication 33.

35. Bactérie-hôte gram-négative recombinante qui est transformée avec un vecteur recombinant sur lequel est localisée une séquence d'acide nucléique de fusion en liaison opératoire avec un promoteur, comprenant :
(i) un fragment d'acide nucléique codant pour un peptide-signal,
(ii) un fragment d'acide nucléique codant pour le peptide passager et/ou le polypeptide passager à présenter,
(iii) le cas échéant un fragment d'acide nucléique codant pour un site de reconnaissance d'une protéase,
(iv) un fragment d'acide nucléique codant pour un lieur transmembranaire et
(v) un fragment d'acide nucléique codant pour un domaine transporteur d'un autotransporteur ;
le fragment d'acide nucléique (ii) étant hétérologue par rapport au fragment d'acide nucléique codant pour le domaine transporteur (v) et la bactérie-hôte étant homologue par rapport au fragment d'acide nucléique codant pour le domaine transporteur (v).

36. Bactérie-hôte selon la revendication 35, **caractérisée en ce qu'**elle est une cellule d'*E.coli.*

37. Bactérie-hôte selon l'une quelconque des revendications 35 à 36, **caractérisée en ce que** le fragment d'acide nucléique (v) code pour le domaine transporteur de la protéine AIDA ou d'une variante de celle-ci, ladite variante ayant une homologie d'au moins 80% avec le domaine transporteur au moins dans la région des structures feuillets plissés bêta.
